# EUROPEAN PATENT SPECIFICATION

(11) **EP 2 179 052 B1**
(45) Date of publication and mention of the grant of the patent: **14.11.2012**
(21) Application number: 08775898.3
(22) Date of filing: 09.07.2008
(51) Int. Cl.: C12N 9/52, A61K 38/48, C12N 9/00, C12Q 1/04, C12Q 1/68, C12Q 1/25, C12Q 1/02, C12Q 1/18

(54) **DETECTION OF MICRO-ORGANISMS BASED ON THEIR NAD-DEPENDENT DNA LIGASE ACTIVITY**
NACHWEIS VON MIKROORGANISMEN AUF DER GRUNDLAGE IHRER NAD-ABHÄNGIGEN DNA-LIGASE-AKTIVITÄT
DÉTECTION DE MICRO-ORGANISMES, BASÉE SUR LEUR ACTIVITÉ LIGASE NAD-DÉPENDANTE

(30) Priority: 09.07.2007 GB 0713255; 18.07.2007 GB 0713972; 10.10.2007 GB 0719785; 15.02.2008 GB 0802857; 17.04.2008 GB 0807054
(43) Date of publication of application: 28.04.2010
(73) Proprietor: Microsens Medtech Ltd, London NW1 0NH (GB)
(72) Inventor: STANLEY, Christopher, John, Cambridge CB3 9ED (GB); WILSON, Stuart, Upper Norwood London SE19 2UX (GB)
(74) Representative: Spencer, Matthew Peter
(86) International application number: PCT/GB2008/002354
(87) International publication number: WO 2009/007719

(56) References cited:
- WO-A-2004/106547
- WO-A-2005/042778
- WO-A-2005/093089
- DE-A1- 4 033 752
- US-A- 4 931 390
- US-A- 5 976 806
- LIU LINGFENG ET AL: "Using molecular beacon to monitor activity of E. coli DNA ligase." THE ANALYST MAR 2005, vol. 130, no. 3, March 2005 (2005-03), pages 350-357, XP002499297 ISSN: 0003-2654
- SCOTT ET AL: "Immobilized DNA hairpins for assay of sequential breaking and joining of DNA backbones" ANALYTICAL BIOCHEMISTRY, ACADEMIC PRESS INC. NEW YORK, vol. 358, no. 1, 1 November 2006 (2006-11-01), pages 90-98, XP005694526 ISSN: 0003-2697
- CHEN XINYI CYNTHIA ET AL: "Development of a fluorescence resonance energy transfer assay for measuring the activity of Streptococcus pneumoniae DNA ligase, an enzyme essential for DNA replication, repair, and recombination." ANALYTICAL BIOCHEMISTRY 15 OCT 2002, vol. 309, no. 2, 15 October 2002 (2002-10-15), pages 232-240, XP002499830 ISSN: 0003-2697
- MALATESTA M L ET AL: "EGTA INHIBITION OF DNASE ACTIVITY IN COMMERCIAL LYSOSTAPHIN PREPARATIONS" BIOTECHNIQUES, INFORMA LIFE SCIENCES PUBLISHING, WESTBOROUGH, MA, US, vol. 12, no. 1, 1 January 1992 (1992-01-01), page 70,72, XP008101426 ISSN: 0736-6205
- MALATESTA M L ET AL: "NUCLEASE CONTAMINATION OF COMMERCIAL LYSOSTAPHIN PREPARATIONS" ABSTRACTS OF THE GENERAL MEETING OF THE AMERICAN SOCIETY FOR MICROBIOLOGY, THE SOCIETY, WASHINGTON, DC, US, vol. 91, 1 January 1991 (1991-01-01), page 262, XP008101450 ISSN: 1060-2011
- IVERSEN O J ET AL: "Studies on lysostaphin. Separation and characterization of three enzymes." EUROPEAN JOURNAL OF BIOCHEMISTRY / FEBS 5 OCT 1973, vol. 38, no. 2, 5 October 1973 (1973-10-05), pages 293-300, XP002513258 ISSN: 0014-2956
- SZWEDA PIOTR ET AL: "New effective sources of the Staphylococcus simulans lysostaphin." JOURNAL OF BIOTECHNOLOGY 4 MAY 2005, vol. 117, no. 2, 4 May 2005 (2005-05-04), pages 203-213, XP002513259 ISSN: 0168-1656
- SHARMA ET AL: "Cytoplasmic expression of mature glycylglycine endopeptidase lysostaphin with an amino terminal hexa-histidine in a soluble and catalytically active form in Escherichia coli" PROTEIN EXPRESSION AND PURIFICATION, ACADEMIC PRESS, SAN DIEGO, CA, vol. 45, no. 1, 1 January 2006 (2006-01-01), pages 206-215, XP005198200 ISSN: 1046-5928
- WILKINSON A ET AL: "Bacterial DNA ligases.", MOLECULAR MICROBIOLOGY JUN 2001 LNKD- PUBMED:11442824, vol. 40, no. 6, June 2001 (2001-06), pages 1241-1248, XP002586885, ISSN: 0950-382X

### SEQUENCE LISTING

<110> Iseao Technologies Limited
<120> Improved Detection of Microorganisms
<130> P93842WO00
<150> 0713255.8
   <151> 2007-07-09
<150> 0713972.8
   <151> 2007-07-18
<150> 0719785.8
   <151> 2007-10-10
<150> 0802857.3
   <151> 2007-02-15
<150> 0807054.2
   <151> 2007-04-17
<160> 11
<170> Patent In version 3.4
<210> 1
   <211> 44
   <212> DNA
   <213> Artificial sequence
<220>
   <223> Oligo
<400> 1
   gccgatatcg gacaacggcc gaactgggaa ggcgcacgga gaga 44
<210> 2
   <211> 45
   <212> DNA
   <213> Artificial sequence
<220>
   <223> Oligo
<400> 2
   cgtggtctct ccgtgcgcct tcccagttcg gccgttgtcc gatat 45
<210> 3
   <211> 38
   <212> DNA
   <213> Artificial sequence
<220>
   <223> Oligo
<400> 3
   ccacgaagta ctagctggcc gtttgtcacc gacgccta 38
<210> 4
   <211> 33
   <212> DNA
   <213> Artificial sequence
<220>
   <223> Oligo
<400> 4
   taggcgtcgg tgacaaacgg ccagctagta ctt 33
<210> 5
   <211> 23
   <212> DNA
   <213> Artificial sequence
<220>
   <223> Primer
<400> 5
   ggacaacggc cgaactggga agg 23
<210> 6
   <211> 25
   <212> DNA
   <213> Artificial sequence
<220>
   <223> Primer
<400> 6
   taggcgtcgg tgacaaacgg ccagc 25
<210> 7
   <211> 49
   <212> DNA
   <213> Artificial sequence
<220>
   <223> Oligo
<400> 7
   ccccggatcc cttagaattc ccctcagagg cactggagct ggagacgta 49
<210> 8
   <211> 78
   <212> DNA
   <213> Artificial sequence
<220>
   <223> Oligo
<400> 8
   gtgcctctga gccaggggag cagttcggcg tagtgatgac gagtctacga gtctacgagt 60
   tctacgtctc cagctcca 78
<210> 9
   <211> 23
   <212> DNA
   <213> Artificial sequence
<220>
   <223> Primer
<400> 9
   gagtctacga gtctacgagt tct 23
<210> 10
   <211> 20
   <212> DNA
   <213> Artificial sequence
<220>
   <223> Primer
<400> 10
   tcatcactac gccgaactgc 20
<210> 11
   <211> 24
   <212> DNA
   <213> Artificial sequence
<220>
   <223> Probe
<400> 11
   ctcagaggca ctggagctgg agac 24

## Description

### FIELD OF THE INVENTION

The present invention relates to the field of detecting micro-organisms, in particular detection of bacteria. The methods of the invention are highly sensitive and have numerous applications. Methods and kits are described which rely upon a novel indicator of bacterial viability.

### BACKGROUND TO THE INVENTION

Measuring the presence and levels of certain molecules which are associated with cell viability is important in a number of contexts. For example, measuring levels of ATP is useful in mammalian cells for growth analysis and toxicology purposes.

Culture approaches can be used to detect small numbers of bacteria but such techniques require several days to complete, especially when attempting to detect small numbers of bacteria and also when detecting slower growing micro organisms.

Alternatively, tests may be carried out based upon measuring the presence of a molecule which can be linked to the presence in the sample of a contaminant cell or organism. The most commonly detected molecule is Adenosine Triphosphate (ATP). Detection of DNA and RNA has also been proposed, although the correlation between the presence of DNA and RNA and viability is not clear-cut due to the variable persistence of nucleic acids in cells post death (Keer & Birch, Journal of Microbiological Methods 53 (2003) 175-183). Detection of adenylate kinase as an indicator of viability has also been proposed (Squirrell DJ, Murphy MJ, Leslie RL, Green JCD: A comparison of ATP and adenylate kinase as bacterial cell markers: correlation with agar plate counts. In Bioluminescence and chemiluminescence progress and current applications. Edited by: Stanley RA, Kricka LJ. John Wiley and Sons; 2002 and WO 96/02665)

A routinely employed method for determining ATP levels involves the use of bioluminescence. The method uses the ATP dependency of the reaction in which light emitting luciferase catalyzes oxidation of luciferin. The method may be used to measure relatively low concentrations of ATP. Kits useful for detecting ATP using bioluminescence are commercially available from Roche, New Horizons Diagnostics Corp, Celsis etc.

A number of problems exist with respect to bioluminescence detection. For example, detection of microbial ATP only, in the presence of ATP from non-microbial sources can be a problem. This problem has been solved to a certain degree by use of filters which can separate bacteria from non-bacterial sources of ATP, thus providing a more accurate signal.

In addition, chemicals and/or metals in a sample can interfere with the bioluminescence reaction. This is of particular relevance, for example, where surface contamination is being measured following cleaning of a surface using cleaning agents. The chemical cleaning agents interfere with the luciferase catalysed reaction, and thus in some cases lead to false negative results, where microbial or other contaminant ATP is present but the bioluminescence reaction is not effective.

Ligases are enzymes which catalyze ligation of nucleic acid molecules. The ligation reaction requires either ATP or NAD+ as co-factor depending upon the ligase concerned.

WO 2005/093089 describes methods and kits for detecting a group of organisms which share the same enzymatic activity in a sample. The main enzymatic activities which are shown to be useful according to the method of WO 2005/093089 are esterases and especially lipases.

US 5,976,806 relates to a DNA ligase activity assay which relies upon ligation in order to repair a restricted plasmid construct including a reporter gene. Thus, detection of ligase activity relies upon expression of a reporter gene product.

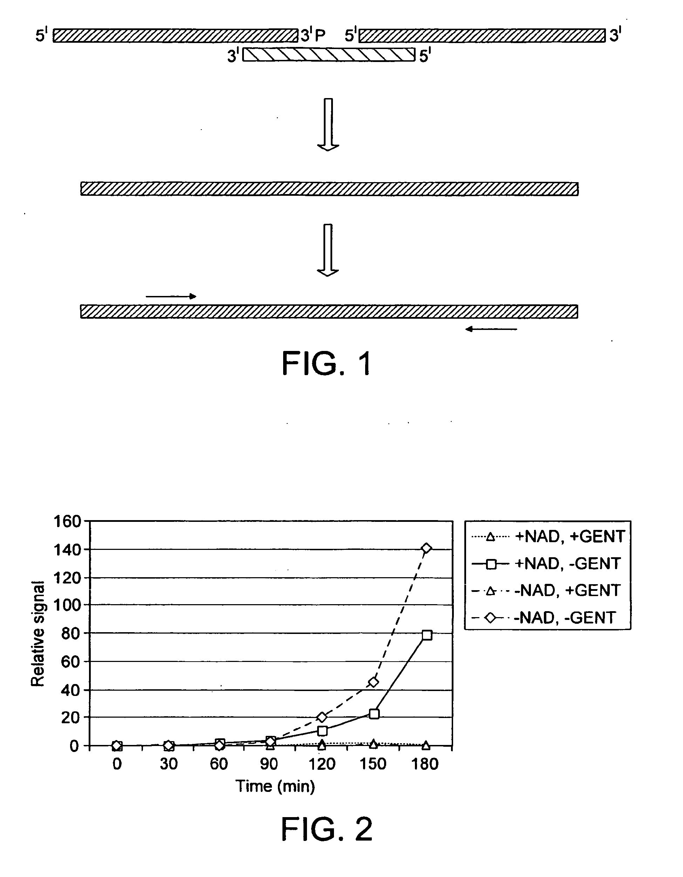

Liu et al., Analyst, 2005, 130, 350-357 discuss using molecular beacons to monitor activity of E. coli DNA ligase. In Liu, as shown in figure 1, the scheme for detection of ligase activity relies upon formation of a double-stranded DNA around a molecular beacon loop in order to separate a donor and acceptor/quencher fluorophore.

Scott et al., Analytical Biochemistry, 358, 2006, 90-98 describe immobilized DNA hairpins for assaying sequential breaking and joining of DNA backbones. In Scott, ligase activity is detected through repair of a nick in a hairpin containing a fluorophore.

Chen et al., Analytical Biochemistry, 309, 2002, 232-240 report on development of a fluorescence resonance energy transfer assay for measuring the activity of *S. pneumoniae* DNA ligase. In Chen, as shown in figure 1 detection of ligase activity is dependent upon two double-stranded DNA molecules being joined which allows fluorescence resonance energy transfer between a donor and acceptor fluorophore.

### SUMMARY OF THE INVENTION

The present invention identifies ligases, in particular NAD-dependent ligases, as a useful indicator of the presence of a (viable) micro-organism or microbe. The invention is defined in the claims.

Accordingly, the invention provides for the use of NAD-dependent ligase activity as an indicator of the presence of a (viable) micro-organism in a sample. The link between NAD-dependent ligase activity and viability is central to the invention (Korycka-Machala et al., Antimicrobial Agents and Chemotherapy, Aug. 2007, p2888-2897) since it allows the activity of this enzyme to be used as an indicator of viable microbial cells, in particular of bacterial origin, in the sample.

Similarly, the invention provides a method of detecting an NAD-dependent ligase as an indicator of the presence of a micro-organism in a sample comprising:
(a) contacting the sample with a ligatable nucleic acid molecule which acts as a substrate for NAD-dependent ligase activity in the sample,
(b) incubating the thus contacted sample under conditions suitable for NAD-dependent ligase activity; and
(c) specifically determining the presence (and/or the amount) of a ligated nucleic acid molecule resulting from the action of the NAD-dependent ligase on the substrate nucleic acid molecule to indicate the presence of the micro-organism; wherein the presence of a ligated mucleic acid molecule is determined using a nucleic acid amplification technique.

Thus, the methods of the invention are useful for identifying all micro-organisms in which an NAD-dependent ligase is (or has been) expressed. The methods may therefore be coined as a method of detecting an NAD-dependent ligase expressing micro-organism in a sample. In certain embodiments, the methods of the invention are applied to the detection of viable micro-organisms and thus may be considered as a method for detecting a viable micro-organism in a sample. In particular, the methods may be useful for identifying bacteria or micro-organisms in which the NAD-dependent ligase gene is essential for viability. However, micro-organisms, such as bacteria, recently rendered non-viable (for example through treatment with an anti-bacterial as discussed herein) may retain detectable NAD-dependent ligase activity until the enzyme is degraded. Thus, reference to micro-organisms may include recently viable micro-organisms, up until the point where NAD-dependent ligase has been degraded, as appropriate. If a distinction between viable and recently viable micro-organisms is required, a simple time course or comparison of NAD-dependent ligase activity between two or more time points, under appropriate conditions, should be sufficient to determine whether NAD-dependent ligase activity increases, persists or diminishes over time. If the NAD-dependent ligase activity persists for, or increases over, an extended period or at (a) later time point(s) (compared to the initial measurement), this may indicate that the micro-organisms are viable. If NAD-dependent ligase activity diminishes at (a) later time point(s), this may indicate that the detected activity was from recently viable micro-organisms. Detection methods are discussed in detail herein. In specific embodiments, the micro-organism is a bacterium. All (eu)bacteria are believed to contain at least one gene encoding an NAD-dependent (DNA) ligase. In a more specific embodiment, the bacterium is a eubacterium. However, NAD-dependent ligases have also been found in thermophilic and cold-resistant bacteria and accordingly, the methods of the invention may be more generally applicable (Wilkinson et al., Molecular Microbiology (2001) 40(6), 1241-1248). Thus, the bacteria may be mesophilic and/or thermophilic bacteria for example.

A "sample" in the context of the present invention is defined to include any sample in which it is desirable to test for the presence of a micro-organism, in particular a bacterium, expressing an NAD-dependent ligase. Thus the sample may comprise, consist essentially of or consist of a clinical sample, or an *in vitro* assay system for example. Samples may comprise, consist essentially of or consist of beverage or food samples or preparations thereof, or pharmaceutical or cosmetic products such as personal care products including shampoos, conditioners, moisturisers etc., all of which are tested for microbial contamination as a matter of routine. The sample may comprise, consist essentially of or consist of tissue or cells and may comprise, consist essentially of or consist of a sputum or a blood sample or a platelet sample for example. In addition, the methods of the invention may be used to monitor contamination of surfaces, such as for example in locations where food is being prepared. Contamination is indicated by the presence of NAD-dependent ligase activity. The contamination may be from any microbial source, in particular bacterial contamination. Furthermore, the invention is also useful in monitoring environmental conditions such as water supplies, wastewater, marine environments etc. The invention is also useful in monitoring bacterial growth in fermentation procedures and in air sampling where bacteria or spore content can be assessed in hospital, industrial facilities or in biodefence applications.

By "NAD-dependent ligase" is meant a DNA ligase which depends upon the nicotinamide adenine dinucleotide (NAD+) cofactor for activity. NAD-dependent ligases can be distinguished from ATP-dependent ligases which rely upon the cofactor ATP for activity. The activity of the NAD-dependent ligase is the formation of a phosphodiester bond between the 5' end of a nucleic acid molecule and the 3' end of a nucleic acid molecule.

The methods of the invention rely on the fact that if there are one or more (viable) micro-organisms, in particular bacteria, present in the sample, the NAD-dependent ligase will be present. This enzyme can thus, under appropriate conditions, catalyse a ligation reaction to generate a novel detectable nucleic acid molecule (in a subsequent process). The novel nucleic acid molecule may be detected by any suitable nucleic acid amplification technique, thereby allowing a determination of the presence of the micro-organisms in the sample under test.

Thus, if the micro-organism is not present in the sample, there will be no NAD-dependent ligase activity in the sample and thus the novel detectable nucleic acid molecule will not be generated.

The methods of the present invention provide significant technical advantages, due in large part to the fact that a novel nucleic acid molecule is generated as part of the method. In the methods of the present invention, unreacted nucleic acid molecule will not contribute to the signal, and as a result no false positive signals should be produced when the methods are carried out.

Furthermore, the method is highly sensitive providing detection of the NAD-dependent ligase present in the sample down to femtogram and possibly even attogram levels. The sensitivity is derived from the fact that every bacterial cell contains thousands of enzyme molecules, and thus each can catalyse multiple ligation events under suitable conditions. Every bacterial cell must produce ligase activity to repair ongoing genomic damage and this essential activity contributes to its usefulness as a marker for the presence of viable microbial cells. Thus unlike PCR approaches, which must target one or a few copies of a gene per cell or use additional steps or reagents to detect ribosomal or messenger RNA, the approach described herein targets the detection of multiple copies of the NAD-dependent ligase per cell in a simple assay format. The sensitivity is further enhanced compared to other approaches in that each copy of the ligase is able to modify multiple (hundreds or thousands) substrate nucleic acid molecules which can each then be detected.

A further advantage over ATP detection techniques is that ATP is common to bacterial, fungi and mammalian cells and it is necessary to differentiate between human and bacterial ATP, for example, when performing a bacterial detection or viability test. By targeting a bacterial specific enzyme, NAD-dependent ligase, there is specificity for the detection of bacteria. Unlike bacterial ligases which require NAD+ as a cofactor, mammalian and fungi ligases have a requirement for ATP. As NAD+ may be provided in the assay, in particular in large molar excess, interference by mammalian and fungal ligases can be avoided.

As stated herein, the first step in the method comprises, consists essentially of or consists of contacting the sample with a nucleic acid molecule which acts as a substrate for NAD-dependent ligase activity in the sample. Any suitable ligatable molecule which can be specifically detected once ligated may be utilised in the methods of the invention.

For the avoidance of doubt, it is hereby stated that the ligated nucleic acid molecule is generally a novel detectable nucleic acid molecule which has a different overall structure to that of the original (substrate) nucleic acid molecule. Thus, the novel detectable nucleic acid molecule may contain additional nucleotides such that the novel nucleic acid molecule may be uniquely identified, for example by amplification utilising primers which can only bind and produce an amplification product using the ligated nucleic acid molecule as a template. However, it may be that only one strand is extended as compared to the (original) substrate nucleic acid molecule, for example the ligase may seal a nick in one strand of a double stranded substrate molecule.

The substrate nucleic acid molecules for use in the methods of the invention, must be of sequence and structure such that the NAD-dependent ligase can act on the molecule to produce a detectable ligated (novel) nucleic acid molecule.

Suitable substrate nucleic acid molecules for use in the invention are set forth as SEQ ID Nos 1 to 4, 7 and 8 and described in more detail in the experimental section below. It is noted that variants of these sequences may be utilised in the present invention. For example, additional flanking sequences may be added. Variant sequences may have at least 90%, at least 91%, at least 92%, at least 93%, at least 94%, at least 95%, at least 96%, at least 97%, at least 98%, or at least 99% nucleotide sequence identity with the nucleotide sequences of the substrate nucleic acid molecules set forth as SEQ ID NOs 1 to 4, 7 and 8. The nucleic acid molecules may incorporate synthetic nucleotide analogues as appropriate or may be RNA or PNA based for example, or mixtures thereof. They may be labelled, such as usin a fluorescent label, or FRET pair, in certain embodiments to facilitate detection. Suitable detection methods are described herein.

"Nucleic acid" is defined herein to include any natural nucleic acid and natural or synthetic analogues that are capable of being ligated by an NAD-dependent ligase in order to generate a ligated (novel detectable) nucleic acid molecule. The ligation reaction may involve either joining of two DNA molecules or sealing a nick in a nucleic acid molecule to produce a detectable ligated nucleic acid molecule for example. Suitable nucleic acid molecules may be composed of, for example, double or single-stranded DNA and double or single-stranded RNA. Nucleic acid molecules which are partially double-stranded and partially single-stranded are also contemplated in certain embodiments of the invention. In certain embodiments the substrate nucleic acid molecule comprises, consists essentially of or consists of dsDNA, to include nicked dsDNA. The term "nucleic acid" encompasses synthetic analogues which are capable of being ligated by NAD-dependent ligase in a sample in an analogous manner to natural nucleic acids, for example nucleic acid analogues incorporating non-natural or derivatized bases, or nucleic acid analogues having a modified backbone. In particular, the term "double-stranded DNA" or "dsDNA" is to be interpreted as encompassing dsDNA containing non-natural bases.

Though the nucleic acid substrate may comprise, consist essentially of or consist of a blunt-ended double-stranded DNA molecule, in a separate embodiment the nucleic acid substrate for the NAD-dependent ligase comprises, consists essentially of or consists of two double stranded DNA molecules with a complementary overhang and 5' phosphate groups at the ends to be joined. In one specific embodiment, the complementary overhang is between 2 and 10, such as 3 or 5 base pairs. In an alternative embodiment, the nucleic acid substrate comprises, consists essentially of or consists of a partially double-stranded DNA molecule with a nick containing a 5' phosphate. Synthesized nucleic acid molecules are commercially available and can be made to order with a terminal 5' phosphate group attached. This has the technical advantage that 100% of the nucleic acid molecules used in the methods of the invention will be labelled with a 5' phosphate group. Furthermore, the nucleic acid substrates can be designed to specification, for example to include biotin molecules for subsequent post-ligation capture if so desired, as described herein.

Thus, in embodiments of the invention, the novel nucleic acid molecule that is detected is generated by ligation of the 3' end of the nucleic acid molecule to the 5' end of a further nucleic acid molecule. In these embodiments, if the ligase is present in the sample, it will catalyse the ligation and a ligated nucleic acid molecule (incorporating an overall novel sequence) will be formed which can be detected by a subsequent process, as detailed herein (a nucleic acid amplification process).

Thus, the substrate nucleic acid molecule may, in fact, comprise, consist essentially of or consist of two or more nucleic acid molecules as appropriate. This applies generally to the methods of the invention.

In certain embodiments, the nucleic acid substrate comprises, consists essentially of or consists of two double stranded nucleic acid molecules with single-stranded complementary overhangs.

The 3' end of nucleic acid substrate molecules that are not productively joined in terms of producing a ligated product which is then detected (desired to be joined) may be blocked with a suitable blocking group in order to ensure that they cannot participate in a ligation reaction. Any appropriate blocking group may be utilised.

In specific embodiments, the nucleic acid molecule which acts as a substrate for NAD-dependent ligase activity in the sample comprises, consists essentially of or consists of a nicked double stranded nucleic acid molecule. In specific embodiments, the overall substrate may be made up of three specific single stranded DNA (ssDNA) molecules. Two or more of the ssDNA molecules may be of identical sequence. One ssDNA molecule may hybridize to the other two nucleic acid molecules in a manner such that a double stranded region is formed that contains a nick. NAD-dependent ligase activity, if present in the sample, may seal the nick thus producing a double stranded DNA molecule which can be detected according to the methods described herein. Suitable examples of such an arrangement include the nucleic acid molecules set forth as SEQ ID No:7 and 8 (two copies), as discussed herein.

In further specific embodiments, the nucleic acid molecule which acts as a substrate for NAD-dependent ligase activity in the sample comprises, consists essentially of or consists of two nucleic acid molecules which can be ligated together.

Preferably, the nucleic acid substrate is present in excess, and in particular in large molar excess, over the ligase in the sample. This is an important technical distinction over prior art methods. Because a novel ligated nucleic acid molecule is detected, only the presence of this molecule in the sample is essential for the detection methods to work effectively. Thus, it is not detrimental to the methods of the invention if other nucleic acid molecules are present in the sample such as from the bacteria to be detected or from mammalian or fungal sources which may be found in the sample to be tested for example.

Preferably, the substrate nucleic acid molecules are designed such that they do not have high levels of homology with the genome of the one or more bacteria or other micro-organisms which produce the NAD-dependent ligase which is to be detected in the sample. This means that, even in the presence of contaminating nucleic acid molecules, only the novel ligated nucleic acid molecule may be detected. Thus, the substrate should have sufficiently low levels of sequence identity with the genomic DNA of the bacteria to be detected to prevent non-specific amplification of genomic DNA producing a false positive result. The sequence of the substrate may thus be designed with the target bacteria in mind. In particular, the primers for amplifying specifically the novel ligated nucleic acid molecule are designed such that they do not produce an amplification product from the bacterial genomic DNA. For example, the substrate and primers may incorporate complementary non-naturally occurring molecules which can base pair with each other, and allow specific amplification of bacterial genomic DNA. As an example, pyDAD and puADA may be incorporated into primers and substrate molecules as appropriate (Sismour et al., Nucleic Acids Research, 2004, Vol. 32, No. 2: 728-735).

Preferably, the homology is less than about 5%, less than about 10%, less than about 12.5 %, less than about 15%,less than about 20%, less than about 30%, less than about 40%, 50%, 60%, 70% or 80% sequence identity with the corresponding nucleotide sequence from the one or more bacteria or other micro-organisms which produce the NAD-dependent ligase which is to be detected in the sample. In one embodiment, there is no sequence identity with the corresponding nucleotide sequence from the one or more bacteria or other micro-organisms which produce the NAD-dependent ligase which is to be detected in the sample over approximately 10, 20, 30, 40 or 50 contiguous nucleotides. In another embodiment, there is less than about 10% or less than about 12.5 %, 15%, 20%, 30%, 40%, 50% or 60% sequence identity over approximately 10, 20, 30, 40 or 50 contiguous nucleotides with the corresponding nucleotide sequence from the one or more bacteria or other micro-organisms which produce the NAD-dependent ligase which is to be detected in the sample.

The second step of the methods of the invention comprises, consists essentially of or consists of incubating the sample under conditions suitable for NAD-dependent ligase activity. Any suitable conditions may be employed, as would be readily determined by one of skill in the art. For example ligation may occur at any temperature between around 4 and 80°C depending upon the ligase concerned (thermophilic bacteria may be detected using reactions incubated at higher temperatures than mesophilic bacteria for example). Preferred incubation temperatures are between around 4 and 40°C, more preferably between around 20 and 37°C and most preferably at room temperature for general (viable) bacterial detection. Suitable incubation times may be between approximately 10 minutes and 10 hours, such as between around 30 minutes, 1 hour or 2 hours and 5, 6, 7, 8 or 9 hours. Incubation may occur in a suitable buffer. Commercially available ligase buffers include *E. coli* ligase buffer available from NEB. Suitable incubation conditions for use of a ligase are well known in the art and are recommended with commercially available ligases.

In specific embodiments, the conditions suitable for NAD-dependent ligase activity include supplying the sample with additional NAD+. The rationale behind this approach of adding NAD+ is that it presents optimal conditions for NAD-dependent ligase activity. Accordingly, NAD-dependent ligase activity in the sample is heavily favoured over any other enzyme activity, such as ATP-dependent ligase activity, in the sample. This is particularly relevant in situations where the sample may contain additional enzyme activity, such as ATP-dependent ligase activity, which could lead to production of a ligated nucleic acid molecule even in the absence of viable bacteria in the sample (i.e. a false positive result). Thus, by actively promoting NAD-dependent ligase activity in the sample, if present, it is possible to identify the presence of viable micro-organisms, in particular bacteria, in the sample even in the presence of potential competing enzyme activity. As is shown in the experimental section below, addition of NAD+ to the sample improves the sensitivity of detection.

In alternative embodiments, no additional NAD+ is added to the sample. Accordingly, here the methods rely upon endogenous NAD+ to support NAD-dependent ligase activity. In the absence of (viable) bacterial cells, there may be no detectable NAD-dependent ligase activity. If bacterial cells are present in the sample, the endogenous NAD+ allows the NAD-dependent ligase to act on the substrate nucleic acid molecule to produce a ligated nucleic acid molecule which is then detected. As shown in the experimental section of the description herein, such methods may permit greater discrimination in signal, since non-viable cells have lower levels of NAD+.

Depending upon the sample type utilised and the particular applications of the methods of the invention, it may be desirable to inhibit any ATP-dependent ligase activity in the sample. In particular, the sample may include eukaryotic cells and/or prokaryotic cells in which an ATP dependent ligase is expressed. Such ATP-dependent ligases may have the ability to act on the substrate nucleic acid molecules to produce a ligated nucleic acid molecule and thus lead to production of false positive results. Accordingly, the methods of the invention may comprise the step of inhibiting ATP-dependent ligase activity in the sample. Inhibition of ATP-dependent ligase activity may be achieved by any suitable means. In one embodiment, ATP-dependent ligase activity is inhibited by depletion of (its cofactor) ATP from the sample. This may be achieved by burning off the ATP in the sample, for example using an enzyme such as luciferase prior to adding the substrate nucleic acid to the sample. ATP-dependent ligase activity may be directly inhibited, for example by adding a competitive substrate. A suitable competitive substrate may comprise, consist essentially of or consist of dATP (Wood WB et al. (1978) J. Biol. Chem. 253, 2437). Incubation conditions may also be altered in order to inhibit ATP-dependent ligase activity without adversely affecting NAD-dependent ligase activity in the sample. For example, use of high concentration of monovalent cations, such as greater than around 200 mM may allow selective inhibition of ATP-dependent ligase activity (Okazaki, R. et al. (1968) Proc. Natl. Acad. Sci USA 59, 598 and Edwards, JB et al. (1991) Nucl Acids Res. 19, 5227).

In further embodiments, the methods of the invention involve capture of any ATP-dependent ligase from the sample prior to determining NAD-dependent ligase activity. This is another way of preventing ATP-dependent activity from influencing the results obtained. The ATP-dependent ligase may be captured from the sample by any suitable means. For example, the ATP-dependent ligase may be captured using a specific reagent, or capture agent, which binds to the ATP-dependent ligase. The binding may be selective for ATP-dependent ligases and/or ATP-dependent ligases from a specific source as appropriate. In a specific embodiment, the ATP-dependent ligase is captured from the sample using an immobilized nucleic acid molecule. Alternative capture agents include antibodies (and derivatives and variants thereof as defined herein), lectins, receptors etc.

Reagents for capture of a ligase (ATP or NAD-dependent, as appropriate) may be immobilised on a solid support in one embodiment. Any suitable solid support may be employed. The nature of the solid support is not critical to the performance of the invention provided that the ligase may be effectively immobilized thereon (without adversely affecting enzyme activity in the case of NAD-dependent forms). Non-limiting examples of solid supports include any of beads, such as polystyrene beads and derivatives thereof and paramagnetic beads, affinity columns, microtitre plates etc. Biotin and streptavidin may be utilised to facilitate immobilisation as required. Methods of immobilization are well known in the art and discussed herein.

In specific embodiments, the methods of the invention further comprise lysis of micro-organisms/bacteria in the sample to release NAD-dependent ligase. The lysis is preferably selective and thus leaves non micro-organism and in particular non-bacterial cells intact. This facilitates detection of NAD-dependent ligase activity in the sample. This step is preferably carried out before the sample is contacted with the nucleic acid substrate, although this is not essential. Suitable agents for lysing bacterial cells selectively are known in the art and include bacterial protein extraction reagents such as B-PER(Pierce) for example. Other conditions may include sonication or French Press for example. However, lysis may not be essential in all embodiments of the invention. In particular, increasing the permeability of the bacterial cell wall and/or membrane may in certain embodiments be sufficient to enable detection of NAD-dependent ligase activity according to the methods of the invention. Suitable agents and techniques for achieving this increase in permeability are known in the art.

A further agent useful in various applications of the present invention is lysostaphin (AMBI). Lysostaphin is an endopeptidase specific for the cell wall peptidoglycan of staphylococci and thus can be used to specifically lyse staphylococcal cells.

The lysostaphin gene has been cloned and can be produced recombinantly. However, the recombinant form commercially available (under the trade name AMBICIN^{®} L) has been found to contain a number of significant impurities relevant to the present invention. In particular, commercially available recombinant lysostaphin includes both nuclease and ligase activity. This is detrimental to the performance of the present invention which relies upon detection of NAD-dependent ligase activity on a nucleic acid based substrate.

In order to overcome the problems with the commercially available lysostaphin, the inventors have devised a process which deactivates the ligase and/or nuclease activity in the preparation whilst retaining the endopeptidase activity of the lysostaphin. Accordingly, described herein is a lysostaphin preparation which is substantially free from nuclease and/or ligase contaminants (and which retains endopeptidase activity). Also described herein is a method for producing a lysostaphin preparation which is substantially free from nuclease and/or ligase contaminants comprising heating a lysostaphin preparation which contains nuclease and/or ligase contaminants under conditions whereby nuclease and/or ligase activity is inhibited and/or reduced whereas (endopeptidase) activity of the lysostaphin is substantially unaffected. Accordingly, further described is a lysostaphin preparation which is substantially free from nuclease and/or ligase contaminants produced by heating a lysostaphin preparation which contains nuclease and/or ligase contaminants under conditions whereby nuclease and/or ligase activity is inhibited and/or reduced whereas (endopeptidase) activity of the lysostaphin is substantially unaffected.

The heating may be to a temperature of between around 50 and 60°C. The lysostaphin preparation may be heated to a temperature of around 55°C since this temperature has been shown to be particularly effective in terms of removing contaminant ligase and/or nuclease activity, whilst retaining (endopeptidase) activity of the lysostaphin.

The preparation may be heated for a suitable period of time. For example, the preparation may be heated for a period of between 1 and 30 minutes such as between 5 and 20 minutes. Longer treatment times may be desirable where lower heating temperatures are employed and vice versa. A particularly suitable treatment is carried out for around 5 minutes. This may be at around 55°C.

Lysostaphin may be included in a suitable ligase buffer. Such a ligase buffer is particularly useful and may include standard components such as salts, detergents, proteins etc. One specific example is described in the experimental section herein and includes MgCl₂, DTT, BSA, NAD+ and Tris (pH8). Thus, also described is a buffer containing NAD+ and lysostaphin, in particular a lysostaphin as described herein. Supplementing the buffer with NAD+ has benefits in terms of promoting NAD-dependent ligase activity as discussed herein.

In further embodiments, the methods of the invention involve capture of any NAD-dependent ligase from the sample. This allows concentration of NAD-dependent ligase activity from the sample and may also allow removal of any inhibitors of enzyme activity which may be found in the sample (through washing for example). The NAD-dependent ligase may be captured from the sample by any suitable means. It is preferred that the NAD-dependent ligase is captured using a specific reagent which binds to the NAD-dependent ligase. The binding may be selective for NAD-dependent ligases and/or NAD-dependent ligases from a specific source as appropriate. In specific embodiments, the NAD-dependent ligase is captured from the sample using an immobilized nucleic acid molecule. More specifically, the immobilized nucleic acid molecule may be the nucleic acid molecule which acts as substrate for NAD-dependent ligase activity in the sample.

In further embodiments the bacterial ligase can be captured from the sample using the nucleic acid substrate immobilized onto a solid surface. For example, in one method the DNA substrate could be immobilized to streptavidin beads through a biotin at the 3' end of one or more of the DNA strands forming the nucleic acid substrate. Any bacterial ligase in a given solution can covalently bind to the 3' hydroxyl of the immobilized DNA substrate and can be captured for subsequent ligation. The advantage of this embodiment is that the ligase can be captured and concentrated from a large volume of solution which can enhance sensitivity of detection. Thus, the methods of the invention may advantageously be utilised to identify or detect bacteria in large volume and/or diluted samples. In addition, the ligase is purified from the bacterial extract which may enhance the activity of the enzyme. Where the substrate nucleic acid molecule comprises of two or more nucleic acid molecules, each of the molecules may be immobilized as appropriate. Each may be immobilized on the same or a separate solid surface as desired.

In other embodiments, the NAD-dependent ligase may be captured using a reagent which may be protein and/or nucleic acid based for example. The reagent may be an antibody, a lectin, a receptor and/or a nucleic acid based molecule. The term "antibody" incorporates all derivatives and variants thereof which retain antigen (NAD-dependent ligase) binding capabilities. Both monoclonal and polyclonal antibodies may be utilised. Derivatised versions, which may be humanized versions of non-human antibodies for example, are also contemplated. Derivatives include, but are not limited to, heavy chain antibodies, single domain antibodies, nanobodies, Fab fragments, scFv etc.

Due to the fact that NAD-dependent ligases share high levels of homology with one another whilst being dissimilar to ATP-dependent ligases (Wilkinson et al., Molecular Microbiology (2001) 40(6), 1241-1248), use of an NAD-dependent ligase specific reagent to capture NAD-dependent ligase represents one useful way of accounting for any ATP-dependent ligase activity which could feasibly be present in the original sample. Moreover, due to the high homology levels, it should also be possible to utilise a generic reagent which can capture NAD-dependent ligase generally. This may be any kind of reagent as discussed above, but is preferably a nucleic acid based reagent, such as a ligatable substrate, or an antibody or derivative thereof. For example, the antibody may be specifically raised against antigens incorporating the conserved motifs from NAD-dependent ligase amino acid sequences (see figure 2 of Wilkinson et al., Molecular Microbiology (2001) 40(6), 1241-1248 for example).

In further embodiments, depending upon the application to which the methods of the invention are put, it may be desirable to distinguish the source of the NAD-dependent ligase. According to such embodiments, a reagent of sufficient specificity is utilised to capture the NAD-dependent ligase of interest. Any specific reagent, which may be protein or nucleic acid based may be utilised. Examples include lectins, receptors, antibodies, DNA and RNA. For example, antibodies may be raised against antigens taken from the non-conserved regions of the NAD-dependent ligase of interest - especially since the amino acid sequence of many ligases are in the public domain (see figure 2 of Wilkinson et al., Molecular Microbiology (2001) 40(6), 1241-1248 for example).

Reagents for capture of an NAD-dependent ligase may be immobilised on a solid support in one embodiment. Any suitable solid support may be employed. The nature of the solid support is not critical to the performance of the invention provided that the NAD-dependent ligase may be immobilized thereon without adversely affecting enzyme activity. Non-limiting examples of solid supports include any of beads, such as polystyrene beads and derivatives thereof and paramagnetic beads, affinity columns, microtitre plates etc. Biotin and streptavidin may be utilised to facilitate immobilisation as required.

Similarly, immobilization chemistry is routinely carried out by those skilled in the art. Any means of immobilization may be utilised provided that it does not have an adverse effect on the methods of the invention, especially in terms of specificity and sensitivity of detection of the ligated nucleic acid molecule produced as an indicator of the presence of a viable micro-organism in the sample.

Once the NAD-dependent ligase has been captured, the immobilised enzyme may be washed to remove inhibitory materials that may affect the subsequent detection process. Thus, in certain embodiments, the method further comprises, consists essentially of or consists of a washing step prior to detection of the novel (ligated) nucleic acid molecule. Washing may utilise any suitable buffer or wash solution, and may include components such as EDTA and Tris-HCl. Suitable wash solutions are well known to those of skill in the art.

In other embodiments, the methods of the invention comprise, as a preliminary step, specific capture of the micro-organism, and in particular bacterium, from a starting sample to produce a test sample in which only the specific bacterium of interest is present. In certain embodiments, the invention provides for a method in which the sample is initially filtered in order to concentrate the one or more bacterial cells or other micro-organisms (as described above). More specific detection of certain bacterial cells or micro-organisms may require specific filtration in order to separate these cells from other (NAD-dependent or ATP dependent) ligase producing cells. Such filters and filtration systems and methods are well known in the art and commercially available (for example from New Horizons Diagnostics Inc.). Specific capture may also be achieved via a suitable reagent. Any specific reagent, which may be protein or nucleic acid based may be utilised. Examples include lectins, receptors, antibodies (and derivatives thereof), DNA and RNA, as discussed herein, which discussion applies *mutatis mutandis*.

The methods of the invention may involve purification of the (novel) ligated nucleic acid molecule prior to detection. Any suitable purification technique may be employed, as are well known in the art. For example nucleic acid may be isolated and run on a gel and the product of the expected size purified prior to detection. Immobilisation of the substrate nucleic acid molecule as described herein may also facilitate purification of the ligated nucleic acid molecule since the ligated nucleic acid molecule will also be immobilized.

In certain embodiments, unligated substrate molecules are selectively removed from the sample or otherwise modified (to prevent their detection) prior to the detection of the ligated nucleic acid molecule. This may be carried out to prevent unligated substrate influencing the sensitivity and/or specificity of detection of the ligated nucleic acid molecules. In specific embodiments, this is achieved by a treatment step employing selective nucleases. In particular, one or more exonucleases may be employed to remove unligated substrate molecules. In specific embodiments, 3'-5' exonucleases such as ExoIII, ExoI and/or ExoT are employed to digest unligated substrate molecules. By controlling the incubation conditions, in particular the temperature and time period of incubation, unligated substrates may be digested but ligated nucleic acid molecules (combining two or more ligated substrates) remain in tact so that they may be detected, especially at the ligation boundary as discuss herein. A combination of dsDNA specific 3'-5' exonucleases, such as ExoIII and one or more ssDNA specific 3'-5' exonucleases such as ExoI and/or ExoT may advantageously be employed where the ligated nucleic acid molecule is formed from at least three substrate nucleic acid molecules which together form a double stranded region including a nick which is ligated by NAD-dependent ligase activity in the sample. The dsDNA 3'-5' exonuclease may act to digest the dsDNA portion to produce ssDNA. If the nick has not been sealed by NAD-dependent ligase, the ssDNA 3'-5' exonuclease may then begin digestion at the ligation site thus removing unligated substrate that could potentially influence the detection reaction. ExoIII may be particularly useful since it does not initiate efficiently at 3' overhangs (see molecular Cloning - A Laboratory Manual, Third Edition, Sambrook and Russell (2001) (Cold Spring Harbour Laboratory Press). By appropriate substrate design, such as using the substrates described herein, such as those comprising, consisting essentially or consisting of the nucleotide sequences set forth as SEQ ID Nos 7 and 8, efficient and specific removal of unligated substrate molecules may be achieved.

Nuclease treatment may be carried out for a suitable period of time such as between 10 minutes and 1 hour, in particular for 30 minutes, and at a suitable temperature such as between 20 and 40°C, in particular at 37°C. The nucleases may then be inactivated to prevent digestion of ligated nucleic acid molecules. Any suitable conditions may be employed. For example, nucleases may be inactivated by treatment at an elevated temperature, such as over 60°C or between 50°C and 100°C, in particular at 95°C. This may be carried out for any appropriate amount of time, such as for 2 to 10 minutes, in particular around 5 minutes.

The methods of the invention may incorporate suitable controls. This may be useful in conjunction with certain sensitive detection techniques, such as nucleic acid amplification techniques (as described herein) to ensure that accurate results have been obtained. For example, the controls may incorporate testing a sample in which NAD-dependent ligase activity is known to be present. If no ligated nucleic acid molecule is produced when the substrate is added to this sample, it is clear there is a problem for example with the reagents used in the methods or with the detection technique. A suitable negative control may be a sample in which there is known to be no NAD-dependent ligase activity. Again, a positive result/detection of similar levels of product as are found in the test sample is an indication that there is a problem. A control in which no nucleic acid based substrate molecule is added may also be employed to ensure the methods are not detecting an unrelated ligation event. All combinations and permutations of appropriate controls are envisaged in the present invention. Suitable controls for use in nucleic acid amplification reactions are employed in specific embodiments of the invention, as described herein.

In preferred embodiments of the invention, the novel nucleic acid molecule, produced according to the presence of NAD-dependent ligase activity in the sample (as an indicator of the presence of one or more (viable) micro-organisms, in particular bacteria in the sample), is detected using nucleic acid amplification techniques.

This serves to make the methods of the invention maximally sensitive. Such amplification techniques are well known in the art, and include methods such as PCR, NASBA (Compton, 1991), 3SR (Fahy et al., 1991), Rolling circle replication, Transcription Mediated Amplification (TMA), strand displacement amplification (SDA) (clinical Chemistry 45: 777-784, 1999), the DNA oligomer self-assembly processes described in US6261846, ligase chain reaction (LCR) (Barringer et al., 1990), selective amplification of target polynucleotide sequences (US 6410276), arbitrarily primed PCR (WO 90/06995), consensus sequence primed PCR (US 4437975), invader technology, strand displacement technology and nick displacement amplification (WO 2004/067726). The list above is not intended to be exhaustive. Any nucleic acid amplification technique may be used provided the appropriate nucleic acid product is specifically amplified.

Amplification is achieved with the use of amplification primers specific for the sequence of the novel/ligated nucleic acid molecule which is to be detected. In order to provide specificity for the nucleic acid molecules primer binding sites corresponding to a suitable region of the sequence may be selected. The skilled reader will appreciate that the nucleic acid molecules may also include sequences other than primer binding sites which are required for detection of the novel nucleic acid molecule produced by the NAD-dependent ligase activity in the sample, for example RNA Polymerase binding sites or promoter sequences may be required for isothermal amplification technologies, such as NASBA, 3SR and TMA.

One or more primer binding sites may bridge the ligation boundary of the substrate nucleic acid molecule such that an amplification product is only generated if ligation has occurred, for example. Alternatively, primers may bind either side of the ligation boundary and direct amplification across the boundary such that an amplification product is only generated (exponentially) if the ligated nucleic acid molecule is formed. As discussed above, primers and the substrate nucleic acid molecule(s) may be designed to avoid non-specific amplification of bacterial genomic DNA.

Suitable primers for use in the methods of the invention are set forth as SEQ ID Nos 5 and 6 or 9 and 10 respectively and described in more detail in the experimental section below. These primers form a separate aspect of the invention. It is noted that variants of these sequences may be utilised in the present invention. In particular, additional sequence specific flanking sequences may be added, for example to improve binding specificity, as required. Variant sequences may have at least 90%, at least 91%, at least 92%, at least 93%, at least 94%, at least 95%, at least 96%, at least 97%, at least 98%, or at least 99% nucleotide sequence identity with the nucleotide sequences of the primers set forth in SEQ ID NOs 5 and 6 or 9 and 10. The primers may incorporate synthetic nucleotide analogues as appropriate or may be RNA or PNA based for example, or mixtures thereof. The primers may be labelled, such as with fluorescent labels and/or FRET pairs, depending upon the mode of detection employed.

Probes may be utilised, again which may be labelled, as desired.

Thus, in certain aspects, the methods of the invention are carried out using nucleic acid amplification techniques in order to detect the novel nucleic acid molecule produced as a direct result of the action of NAD-dependent ligase activity on the substrate nucleic acid molecule which indicates the presence of a bacterial cell or other NAD-dependent ligase expressing micro-organism in the sample. In certain embodiments the technique used is selected from PCR, NASBA, 3SR, TMA, SDA and DNA oligomer self-assembly. Detection of the amplification products may be by routine methods, such as, for example, gel electrophoresis but is preferably carried out using real-time or end-point detection methods.

A number of techniques for real-time or end-point detection of the products of an amplification reaction are known in the art. These include use of intercalating fluorescent dyes such as SYBR Green I (Sambrook and Russell, Molecular Cloning - A Laboratory Manual, Third edition), which allows the yield of amplified DNA to be estimated based upon the amount of fluorescence produced. Many of the real-time detection methods produce a fluorescent read-out that may be continuously monitored; specific examples including molecular beacons and fluorescent resonance energy transfer probes. Real-time and end-point techniques are advantageous because they keep the reaction in a "single tube". This means there is no need for downstream analysis in order to obtain results, leading to more rapidly obtained results. Furthermore keeping the reaction in a "single tube" environment reduces the risk of cross contamination and allows a quantitative output from the methods of the invention. This may be particularly important in the context of the present invention where health and safety concerns may be of paramount importance (such as in detecting potential bacterial contamination of platelet samples for example).

Real-time and end-point quantitation of PCR reactions may be accomplished using the TaqMan^{®} system (Applied Biosystems), see Holland et al; Detection of specific polymerase chain reaction product by utilising the 5'-3' exonuclease activity of Thermus aquaticus DNA polymerase; Proc. Natl. Acad. Sci. USA 88, 7276-7280 (1991), Gelmini et al. Quantitative polymerase chain reaction-based homogeneous assay with flurogenic probes to measure C-Erb-2 oncogene amplification. Clin. Chem. 43, 752-758 (1997) and Livak et al. Towards fully automated genome wide polymorphism screening. Nat. Genet. 9, 341-342 (1995). This type of probe may be generically referred to as a hydrolytic probe. Suitable hydrolytic/Taqman probes for use in real time or end point detection are also provided. They may comprise, consist essentially of or consist of the nucleotide sequence set forth as SEQ ID NO: 11. The probe is suitably labelled, for example using the labels detailed below.

In the Molecular Beacon system, see Tyagi & Kramer. Molecular beacons - probes that fluoresce upon hybridization. Nat. Biotechnol. 14, 303-308 (1996) and Tyagi et al. Multicolor molecular beacons for allele discrimination. Nat. Biotechnol. 16, 49-53 (1998), the beacons are hairpin-shaped probes with an internally quenched fluorophore whose fluorescence is restored when bound to its target. These probes may be referred to as hairpin probes.

A further real-time fluorescence based system which may be incorporated in the methods of the invention is Zeneca's Scorpion system, see Detection of PCR products using self-probing amplicons and fluorescence by Whitcombe et al. Nature Biotechnology 17, 804 - 807 (01 Aug 1999). Additional real-time or end-point detection techniques which are well known to those skilled in the art and which are commercially available include Lightcycler^{®} technology, Amplifluour^{®} primer technology, DzyNA primers (Todd et al., Clinical Chemistry 46:5, 625-630 (2000)), or the Plexor™ qPCR and qRT-PCR Systems.

Thus, in further aspects of the invention the products of nucleic acid amplification are detected using real-time or end point techniques. In specific embodiments of the invention the real-time technique consists of using any one of hydrolytic probes (the Taqman^{®} system), FRET probes (Lightcycler^{®} system), hairpin primers (Amplifluour^{®} system), hairpin probes (the Molecular beacons system), hairpin probes incorporated into a primer (the Scorpion^{®} probe system), primers incorporating the complementary sequence of a DNAzyme and a cleavable fluorescent DNAzyme substrate (DzYNA), Plexor qPCR and oligonucleotide blocking systems.

In certain embodiments, the reaction mixture will contain all of; the sample under test, the substrate nucleic acid molecule(s), reagents, buffers and enzymes required for amplification of the novel (ligated) nucleic acid molecule optionally in addition to the reagents required to allow real time or end-point detection of amplification products. Thus the entire detection method for the NAD-dependent ligase (from the one or more bacterial cells or micro-organisms of interest) may occur in a single reaction, with a quantitative output, and without the need for any intermediate washing steps. Use of a "single tube" reaction is advantageous because there is no need for downstream analysis in order to obtain results, leading to more rapidly obtained results. Furthermore keeping the reaction in a "single tube" environment reduces the risk of cross contamination and allows a quantitative output from the methods of the invention. Also, single tube reactions are more amenable to automation, for example in a high throughput context.

Alternatively, the methods of the invention may be carried out in step-wise fashion. Thus, in a first step it may first be necessary to prepare the sample in a form suitable for use in the method of the invention. For example, as discussed herein, selective cell lysis or increasing cellular permeability may be required. Capture of NAD-dependent ligase may also be desirable again as described herein. ATP dependent ligase activity may be inhibited etc.

The methods of the present invention have a number of applications. For example, they have utility in the important field of microbial resistance to existing treatments. Many bacteria are now resistant to a large number of currently available antimicrobial treatments, and certain strains, such as Methicillin Resistant *Staphylococcus* aureus (MRSA) pose dangerous health risks particularly in a clinical context.

There is, therefore, a requirement for techniques which allow resistance to anti-microbial agents to be readily determined.

Therefore, according to a further aspect, the invention provides a method of screening for resistance of a bacterial cell or other micro-organism to an agent directed against said cell, bacterium or other micro-organism, the method comprising, consisting essentially of or consisting of the steps of, in a sample:
(a) exposing the bacterial cell or micro-organism to the agent;
(b) contacting the sample with a ligatable nucleic acid molecule which acts as substrate for NAD-dependent ligase activity in the sample,
(c) incubating the thus contacted sample under conditions suitable for NAD-dependent ligase activity; and
(d) specifically detecting whether there is present (and/or the amount of) a (novel) ligated nucleic acid molecule resulting from the action of the NAD-dependent ligase on the substrate nucleic acid molecule wherein if there is resistance, the (novel) ligated nucleic acid molecule will be detected (or will be detected at higher levels); wherein the presence of a ligated nucleic acid molecule is determined using a nucleic acid amplification technique.

The same method may also be used as a compound screening method to determine if a new compound, agent or molecule has effect against a particular one or more target bacterial cells or other micro-organisms expressing NAD-dependent ligase as an essential enzyme.

Thus, in a still further aspect the invention provides a method of screening for agents which are capable of killing or preventing growth of one or more bacterial cells or other appropriate micro-organisms, the method comprising, consisting essentially of or consisting of the steps of, in a sample:
(a) exposing the bacterial cell or micro-organism to the agent;
(b) contacting the sample with a ligatable nucleic acid molecule which acts as substrate for NAD-dependent ligase activity in the sample,
(c) incubating the thus contacted sample under conditions suitable for NAD-dependent ligase activity; and
(d) specifically detecting whether there is present (and/or the amount of) a (novel) ligated nucleic acid molecule resulting from the action of the NAD-dependent ligase on the substrate nucleic acid molecule, wherein if the agent is capable of killing or preventing growth of the bacterium or micro-organism the novel nucleic acid molecule will not be detected (or will be detected at lower levels) ; wherein the presence of a ligated mucleic acid molecule is determined using a nucleic acid amplification technique.

This aspect of the invention may also be utilised as a rapid viability test in compound screening. Thus, a compound, agent or molecule may be screened according to the method to determine whether it is toxic to appropriate cells. Thus, a positive result in the method in terms of detecting the novel nucleic acid molecule would indicate that the compound is of low toxicity to the cells. The method of the invention may also prove to have diagnostic utility, whereby an infection may be specifically and sensitively detected in the early stages when only minimal levels of the infecting bacterial cell or other micro-organism expressing an NAD-dependent ligase are present.

Therefore, in a further aspect there is provided a method of diagnosing an infection, or a disease associated with the presence of a bacterial cell or other micro-organism in a subject, comprising, consisting essentially of or consisting of the steps of, in a sample obtained from the subject:
(a) contacting the sample with a ligatable nucleic acid molecule which acts as a substrate for NAD-dependent ligase activity in the sample,
(b) incubating the thus contacted sample under conditions suitable for NAD-dependent ligase activity; and
(c) specifically determining the presence (and/or the amount of) of a ligated nucleic acid molecule resulting from the action of the NAD-dependent ligase on the substrate nucleic acid molecule to indicate the presence of the (viable) bacterium or other micro-organism as an indication of infection or disease; wherein the presence of a ligated mucleic acid molecule is determined using a nucleic acid amplification technique.

In this context the "sample" will generally be a clinical sample. The sample being used will depend on the condition that is being tested for. Typical samples which may be used, but which are not intended to limit the invention, include whole blood, serum, plasma, platelet and urine samples etc. taken from a patient, most preferably a human patient.

The test will be an *in vitro* test carried out on a sample removed from a subject.

Methods of obtaining a suitable sample from a subject are well known in the art. The method may be carried out beginning with a sample that has already been isolated from the patient in a separate procedure. The diagnostic methods will most preferably be carried out on a sample from a human, but the method of the invention may have diagnostic utility for many animals.

The diagnostic methods of the invention may be used to complement any already available diagnostic techniques, potentially as a method of confirming an initial diagnosis. Alternatively, the methods may be used as a preliminary diagnosis method in their own right, since the methods provide a quick and convenient means of diagnosis. Furthermore, due to their inherent sensitivity, the diagnostic methods of the invention require only a minimal sample, thus preventing unnecessary invasive surgery. Also, a large but non-concentrated sample may also be tested effectively according to the methods of the invention.

Thus, the methods of the invention have multiple applications beyond detection of contaminating organisms in a sample. The description provided above with respect to the first aspect of the invention applies *mutatis mutandis* to the further aspects of the invention and is not repeated for reasons of conciseness. For example, suitable controls may be incorporated for these methods of the invention.

In specific embodiments the NAD-dependent ligase is derived from a pathogenic micro-organism, in particular a pathogenic bacterium.

The bacterium may be any bacterium which is capable of causing infection or disease in a subject, preferably a human subject. In one embodiment, the bacteria comprises or consists essentially of or consists of any one or more of Staphylococcus species, in particular Staphylococcus aureus and preferably methicillin resistant strains, Enterococcus species, Streptococcus species, Mycobacterium species, in particular Mycobacterium tuberculosis, Vibrio species, in particular Vibrio cholerae, Salmonella and/or Escherichia coli etc. The bacteria may comprise, consist essentially of or consist of Clostridium species and in particular C. difficile in certain embodiments. C. difficile is the major cause of antibiotic-associated diarrhoea and colitis, a healthcare associated intestinal infection that mostly affects elderly patients with other underlying diseases.

In certain embodiments, according to these further aspects of the invention, the molecule which is being tested in the method (either for resistance or ability to treat an infection or toxicity to cells) is an antimicrobial compound. In the compound screening methods, any molecule may be tested. Examples include antimicrobial agents, nucleic acid molecules including siRNA (dsRNA) molecules and antisense molecules, small molecules, antibodies and all derivatives thereof including Fab fragments, variable region fragments and single domain antibodies for example provided they retain binding affinity etc. The method may be carried out in a high throughput context to screen large numbers of molecules in a short period of time.

The antimicrobial agent, in one embodiment, may be taken from the two main types of antimicrobial agents, antibiotics (natural substances produced by micro-organisms) and chemotherapeutic agents (chemically synthesized), or may be a hybrid of the two such as semi-synthetic antibiotics (a subsequently modified naturally produced antibiotic) or synthetic antibiotics (synthesised versions of natural antibiotics).

Suitable candidate antimicrobial agents may, following a positive result in the methods of the invention in terms of ability to kill or prevent growth of a bacterium or bacterial cell or other suitable micro-organism be tested for at least one or more of the following properties:
(1) the agent should be non-toxic to the subject and without adverse side effects,
(2) the agent should be non-allergenic to the subject,
(3) the agent should not eliminate the natural flora of the subject,
(4) the agent should be stable,
(5) the agent should preferably be cheap and readily available/easy to manufacture; and
(6) the agent should be sufficiently potent that pathogen resistance does not develop (to any appreciable degree). This feature may be tested according to the methods described above.

In one embodiment, a combination of multiple suitable antimicrobial agents may be tested for ability to treat an infection and/or for resistance thereto.

Antibiotics or derivatives thereof which may be tested for resistance and perhaps also for their novel ability to treat certain infections may be selected from the following groups, provided by way of example and not limitation; beta-lactams such as penicillin, in particular penicillin G or V, and cephalosporins such as cephalothin, semi-synthetic penicillins such as ampicillin, methicillin and amoxicillin, clavulanic acid preferably used in conjunction with a semi-synthetic penicillin preparation (such as clavamox or augmentin for example), monobactams such as aztreonam, carboxypenems such as imipenem, aminoglycosides such as streptomycin, kanamycin, tobramycin and gentamicin, glycopeptides such as vancomycin, lincomycin and clindamycin, macrolides such as erythromycin and oleandomycin, polypeptides such as polymyxin and bacitracin, polyenes such as amphotericin and nystatin, rifamycins such as rifampicin, tetracyclines such as tetracycline, semi-synthetic tetracyclines such as doxycycline, chlor tetracycline, chloramphenicol, quinolones such as nalidixic acid and fluoroquinolone and competitive inhibitors such as sulfonamides, for example gantrisin and trimethoprim. Ceftriaxone and/or nitroflurazone may also be utilised.

The methods of the invention may also be applied to determining the presence of bacteria of interest which are resistant to a designated or specific anti-bacterial agent. Accordingly, the present invention provides a method for determining the presence of bacteria of interest, which are resistant to a specific anti-bacterial agent, in a sample comprising, consisting essentially of or consisting of (the following steps, in particular in the order specified):
(a) incubating the bacteria of interest in an incubating medium including the specific anti-bacterial agent,
(b) capturing the incubated bacteria of interest using a specific capture reagent,
(c) exposing the captured bacteria of interest to an agent capable of causing lysis of the bacteria or of increasing the permeability of the bacterial cell wall to a degree such that the presence of NAD-dependent ligase from the bacteria can be determined, and
(d) determining the presence of the (resistant) bacteria of interest in the sample by:
   (i) contacting the sample with a nucleic acid molecule which acts as a substrate for NAD-dependent ligase activity in the sample,
   (ii) incubating the thus contacted sample under conditions suitable for NAD-dependent ligase activity; and
   (iii) specifically determining the presence of a ligated nucleic acid molecule resulting from the action of the NAD-dependent ligase on the substrate nucleic acid molecule to indicate the presence of the NAD-dependent ligase expressing micro-organism; wherein the presence of a ligated nucleic acid molecule is determined using a nucleic acid amplification technique.
The order of capture (step (b)) follow by culture (step (a)) is generally preferable since this only requires a single culture step in specific embodiments. Where culture is carried out before capture, it has been found in practice that an additional culture step post capture may be required to ensure sufficient sensitivity in the tests.

The methods of the present invention may include an additional intervening step or steps so as to enable the presence of more than one target bacteria to be determined. Typically, these steps may comprise capturing multiple different bacteria of interest using one or more appropriate capture agents. Similarly, the methods of the invention may include a number of such steps so that the presence of other target bacteria may be determined.

The bacteria of interest may be any bacteria which are known to be resistant to a specific anti-bacterial agent. Typically, the bacteria may be infection or disease causing bacteria which are known to have resistance to one or more specific anti-bacterial agents. In certain embodiments, the bacteria comprises or consists essentially of or consists of any one or more of Staphylococcus species, in particular Staphylococcus aureus and most particularly methicillin resistant strains, Enterococcus species, Streptococcus species, Mycobacterium species, in particular Mycobacterium tuberculosis, Vibrio species, in particular Vibrio cholerae, Salmonella and/or Escherichia coli etc. The bacteria may comprise, consist essentially of or consist of Clostridium species and in particular C. difficile in certain embodiments.

The specific anti-bacterial agent to which the bacteria of interest are resistant may be any specific anti-bacterial agent. The agent is advantageously utilised in step (a) to prevent, inhibit or restrict sensitive bacteria which may have been captured in step (b) from growing. The agent may cause lysis of sensitive bacteria, although that is not essential. The agent may be an antibiotic or a chemotherapeutic agent or may be a hybrid of the two (semi-synthetic or synthesised antibiotic), as discussed herein. Antibiotics or derivatives thereof to which certain bacteria are resistant and which may therefore be employed in the methods of the invention may be selected from those listed herein, which list applies mutatis mutandis.

In certain embodiments, beta-lactam antibiotics such as ampicillin, methicillin and amoxicillin and in particular methicillin are utilised in the methods. Here, the bacteria may be Staphylococcus aureus, in particular the method may be used to detect MRSA in a sample.

In this context the "sample" will generally be a clinical sample. The sample being used may depend on the type of resistant bacteria that is being tested for. Typical samples which may be used, but which are not intended to limit the invention, include nasal, groin and armpit swabs etc. taken from a patient, in particular from a human patient. Generally, the methods of this aspect of the invention will be an *in vitro* test carried out on a sample removed from a subject.

In further embodiments, the above-described methods may additionally include the step of obtaining the sample from a subject. Methods of obtaining a suitable sample from a subject are well known in the art. Alternatively, the method may be carried out beginning with a sample that has already been isolated from the patient in a separate procedure. The methods will generally be carried out on a sample from a human, but the method of the invention may have diagnostic utility for many animals.

Step (b) which may be performed first, is designed to capture the bacteria of interest from the general (clinical) sample, which may include other cell types, such as mammalian cells and other bacterial cells. Any suitable specific capture reagent may be employed for this purpose. In certain embodiments the specific capture reagent may be specific only to a particular species of bacteria. In other embodiments the specific capture reagent may be specific to a particular strain of bacteria. For example, the specific capture reagent may allow capture of Staphylococci generally or it may allow capture of S. aureus more specifically, or even only antibiotic resistant strains, such as MRSA, of S. aureus only. The specific capture reagent may be protein and/or nucleic acid based for example. The reagent may be an antibody, a lectin, a receptor and/or a nucleic acid based molecule for example. The term "antibody" incorporates all derivatives and variants thereof which retain antigen binding capabilities. Both monoclonal and polyclonal antibodies may be utilised. Derivatised versions, which may be humanized versions of non-human antibodies for example, are also contemplated. Derivatives include, but are not limited to, heavy chain antibodies, single domain antibodies, nanobodies, Fab fragments, scFv etc.

In specific embodiments, the specific capture reagent is immobilized on a solid support. Any suitable solid support may be employed. The nature of the solid support is not critical to the performance of the invention provided that the bacteria of interest may be immobilized thereon without adversely affecting growth in the subsequent step. Non-limiting examples of solid supports include any of beads, such as polystyrene beads and derivatives thereof and paramagnetic beads, affinity columns, microtitre plates etc. Biotin and streptavidin may be utilised to facilitate immobilisation as required.

Similarly, immobilization chemistry is routinely carried out by those skilled in the art. Any means of immobilization may be utilised provided that it does not have an adverse effect on the methods of the invention, especially in terms of specificity and sensitivity of detection of the bacteria of interest.

Step (a) is important to prevent, inhibit or otherwise restrict growth of any captured bacteria which are susceptible to the specific anti-bacterial agent but which were captured in step (b). This step effectively ensures that resistant bacteria of interest can be easily detected and discriminated from bacteria which are captured but are susceptible to the specific anti-bacterial agent. Any suitable incubating medium may be employed. In specific embodiments, a liquid medium is used. Suitable media for permitting bacterial growth are well known in the art and commercially available. As discussed, the incubating medium contains the specific anti-bacterial agent to control growth of any susceptible bacteria captured in step (b). This ensures background in the methods of the invention is kept low and helps to improve the sensitivity and specificity of detection of bacteria of interest which are resistant to the specific anti-bacterial agent. Of course, since the bacteria of interest are resistant to the agent they will continue to grow, thus allowing their sensitive and specific detection following lysis or an increase in cellular permeability, in steps (c) and (d).

Step (c) allows the intracellular content of the bacteria of interest to be released, thus permitting the detection of the bacteria of interest in step (d). Any agent capable of causing cell lysis may be utilised. In specific embodiments of the present invention the agent capable of causing cell lysis is selective towards the bacteria of interest. This is not essential, however, due to step (b) which leads to specific capture and step (a) which prevents growth of susceptible bacteria. In certain embodiments, the agent is selective to Staphylococcus aureus. In more specific embodiments, the agent capable of causing cell lysis is lysostaphin. The lysostaphin may be a commercially available lysostaphin or may be a lysostaphin previously disclosed herein , which discussion applies mutatis mutandis.

In other embodiments, the agent capable of causing cell lysis is a bacteriophage. The bacteriophage may be selective to the bacteria of interest. In alternative embodiments, the agent may be a bacteriocin, such as a colicin or a microcin as appropriate. However, lysis may not be essential in all embodiments of the invention. In particular, increasing the permeability of the bacterial cell wall and/or membrane may in certain embodiments be sufficient to enable detection of intracellular material, such as NAD-dependent ligase activity, according to the methods of the invention. Suitable agents and techniques for achieving this increase in permeability are known in the art.

Step (d) incorporates the detection methods of the invention involving use of NAD-dependent ligase as an indicator of the presence of the bacteria of interest. Thus, a suitable nucleic acid molecule substrate can be added to the intracellular material. If resistant bacteria of interest are present in the sample, NAD-dependent ligase activity will be present and the substrate will be ligated to produce a ligated nucleic acid molecule. Specific detection of this ligated nucleic acid molecule provides an indication of the presence of the resistant bacteria of interest in the starting sample. Accordingly, all embodiments of the methods of the invention, as described herein, may be applied to this aspect of the invention.

In specific embodiments, a measurement of NAD-dependent ligase activity is made at the end of step (b)/beginning of step (a). This measurement provides a background signal against which the test signal can be compared to reach a conclusion regarding the significance of the results. It indicates the basal level of the intracellular material, in particular NAD-dependent ligase activity, in the sample prior to the incubation step which is designed to selectively permit growth of bacteria which are resistant to the specific anti-bacterial agent. A marked or significant increase in the measurement, such as NAD-dependent ligase activity, made in step (d) as compared to that made at the end of step (b)/beginning of step (a) is a reliable indicator of the presence of the bacteria of interest which are resistant to the specific anti-bacterial agent. If there is no increase (or indeed a decrease) as compared to the background signal following the incubation (step (a)) and treatment to increase permeability or cause cell lysis (step (c)), this is an indication of an absence of bacteria which are resistant to the specific anti-bacterial agent.

In certain embodiments of the present invention the method further comprises, consists essentially of or consists of a washing step following step (b) to remove any cells or other materials non-specifically associated with the capture reagent. Such steps would be routine for one skilled in the art when dealing with a specific capture procedure.

The steps of the method are not necessarily restricted to the order specified. In particular, step (b) of capturing the bacteria of interest using a specific capture reagent may be carried out following an initial step (a), namely incubating the bacteria of interest in an incubating medium including the specific anti-bacterial agent.

Also described are test kits for performing these methods of the invention. The test kit may be a disposable test kit . Each component of the test kit may be supplied in a separate compartment or carrier, or one or more of the components may be combined - provided that the components can be stably stored together. The test kit incorporates a specific capture agent for capturing the bacteria of interest resistant to a specific anti-bacterial agent. Suitable capture agents are discussed above, which discussion applies mutatis mutandis. A solid support for the specific capture agent might be provided in the kit. The specific capture agent might be supplied pre-loaded onto the solid support. Suitable solid supports and means of immobilization are described herein

The kit may also incorporate the incubating medium for the bacteria of interest. In specific embodiments, the medium incorporates the specific anti-bacterial agent to which the bacteria of interest is resistant. Alternatively, the agent may be supplied separately and added to the medium only when the methods are carried out. Suitable media and specific anti-bacterial agents are described herein (with respect to the corresponding methods), which discussion applies mutatis mutandis. The medium may be supplied in any suitable form, such as in freeze dried form for example.

The kit may also incorporate a suitable agent capable of causing cell lysis of the bacteria of interest or of increasing the permeability of the bacterial cell wall and/or membrane sufficiently to enable detection of intracellular material (in particular NAD-dependent ligase activity) according to the methods of the invention. Suitable agents are discussed above, which discussion applies mutatis mutandis. The agent might be a lysostaphin such as a (functional) lysostaphin described herein in particular a lysostaphin preparation which is substantially free from nuclease and/or ligase contaminants (produced by inactivating these components - as discussed herein).

The kit may also further incorporate components allowing detection of NAD-dependent ligase activity in the sample. Thus, the kits described herein may be combined with the present kits to provide an NAD-dependent ligase linked detection kit for determining the presence of a bacteria of interest resistant to a specific anti-bacterial agent in a sample. The kits may incorporate at least one nucleic acid molecule which acts as a substrate for NAD-dependent ligase activity in the sample. Suitable substrate nucleic acid molecules, which may comprise two or more ligatable nucleic acid molecules, are discussed herein which discussion applies mutatis mutandis. Thus, the substrate nucleic acid molecules may be immobilized on a solid support or may be supplied together with a solid support to allow immobilization thereon .

The kit incorporates reagents necessary for nucleic acid amplification. Employment of nucleic acid amplification techniques allows sensitive detection of the presence of a novel ligated nucleic acid molecule. Suitable techniques and the necessary reagents would be immediately apparent to one skilled in the art. Thus, the kits in particular incorporate suitable primers for specific detection of the ligated nucleic acid molecule - as discussed in greater detail herein. The kits may also incorporate suitable reagents for real-time detection of amplification products.

The kits may incorporate a suitable carrier in which the reactions take place. Advantageously, such a carrier may comprise a multi-well plate, such as a 48 or 96 well plate for example. Such a carrier allows the detection methods to be carried out in relatively small volumes - thus facilitating scale up and minimising the sample volume required.

The kits will typically incorporate suitable instructions. These instructions permit the methods of the invention to be carried out reliably using the kits.

In one specific aspect, the methods of the invention are utilised in order to detect bacterial contamination of a platelet sample.

Thus, there is provided a method of detecting an NAD-dependent ligase as an indicator of the presence of bacterial contamination in a platelet (containing) sample comprising:
(a) contacting the platelet sample with a ligatable nucleic acid molecule which acts as a substrate for NAD-dependent ligase activity in the sample,
(b) incubating the thus contacted sample under conditions suitable for NAD-dependent ligase activity; and
(c) specifically determining the presence of a ligated nucleic acid molecule resulting from the action of the NAD-dependent ligase on the substrate nucleic acid molecule to indicate the presence of the bacterial contamination in the platelet (containing) sample; wherein the presence of a ligated nucleic acid molecule is determined using a nucleic acid amplification technique.

In specific embodiments, the method for detection of bacterial contamination of platelets comprises, consists essentially of or consists of additional substeps. These substeps may include, for example:
(i) lysis of the platelets under conditions that leave the bacterial cells intact. This principally allows selective dependent ligase activity provided by mammalian cells can be removed prior to testing
(ii) concentration of the bacteria (for example by centrifugation to produce a bacterial cell containing pellet)
(iii) lysis of the bacteria or a treatment to increase the permeability of the bacteria to release the NAD-dependent ligase
The description of the various embodiments of the methods of the invention apply *mutatis mutandis* to this specific application and are not repeated for reasons of conciseness.

Also described are kits which enable and are suitable for carrying out the various methods of the invention.

Therefore, described herein is a kit for carrying out one of the methods of the invention comprising, consisting essentially of or consisting of:
(a) at least one ligatable nucleic acid molecule which acts as a substrate for NAD-dependent ligase activity in the sample wherein the at least one nucleic acid molecule is immobilized on a solid support or is provided together with reagents for immobilizing the substrate nucleic acid molecule on the solid support; and
(b) primers for specific detection of a ligated nucleic acid molecule produced by NAD-dependent ligase activity in the sample.

The embodiments presented in respect of the methods of the invention apply *mutatis mutandis* and are not repeated here for reasons of conciseness. Thus, all necessary components and reagents required to carry out the methods of the invention may be incorporated into the kits . In particular, the substrate nucleic acid molecule includes a dsDNA component which can be ligated by an NAD-dependent ligase. Specific examples may be selected from the nucleic acid substrates comprising the nucleotide sequences set forth as SEQ ID NO: 1 to 4 and/or 7 and 8 respectively (as shown in the detailed description herein). The four nucleic acid molecules comprising the nucleotide sequences set forth as SEQ ID NOs 1-4 respectively can form a substrate molecule with a ligatable complementary 5 nucleotide overlap in one aspect of the invention. In a further aspect, the nucleic acid molecules comprising the nucleotide sequences set forth as SEQ ID NOs 7 and 8 can hybridize to form a substrate molecule which contains a double stranded section with a nick.

The kits may also include appropriate filters in order to separate the bacterial cells or other micro-organisms to be detected from other (ligase producing) cells. Such filters and filtration systems and methods are well known in the art and commercially available (for example from New Horizons Diagnostics Inc.). The kits may also incorporate a suitable filter or filtration mechanism or system in order to be able to isolate target cells or organisms prior to determining whether the NAD-dependent ligase activity is present.

Additionally, specific bacterial cells or other micro-organisms may also be selected and/or isolated by utilising specific reagents which can bind to the cells or micro-organisms. Suitable reagents include both protein and nucleic acid based reagents. Examples include antibodies, lectins, receptors, DNA, RNA etc. Thus, the kits of the invention may additionally comprise, consist essentially of or consist of a suitable reagent for isolating the bacterium or bacteria of interest. Antibodies and all derivatives thereof (such as Fab fragments, single domain antibodies and variable region fragments) which retain specific binding affinity are included in the definition of the term "antibody".

As discussed herein, the substrate nucleic acid molecule may be immobilized on a solid support. The immobilization of the substrate nucleic acid molecule on a solid support allows effective capture of the NAD-dependent ligase from the sample. The interaction of the immobilized substrate nucleic acid molecule with the NAD-dependent ligase results in the generation of a novel, ligated nucleic acid molecule. Thus, the kits may further comprise a solid support. The substrate may or may not be provided pre-loaded on the solid support. If it is not pre-immobilized on the solid support, suitable reagents to allow immobilization may be provided in the kit, optionally together with suitable instructions. Reagents to allow immobilization would be well known to one of skill in the art. Any means of immobilization may be utilised provided that it does not have an adverse effect on the implementation of the methods of the invention, especially in terms of specificity and sensitivity of detection of the NAD-dependent ligase from the one or more target bacterial cells or micro-organisms.

Any suitable solid support may be included in the kits . The nature of the solid support is not critical to the performance of the invention provided that the substrate nucleic acid molecule may be immobilized thereon without adversely affecting NAD-dependent ligase activity, including the ability of the enzyme to interact with the nucleic acid molecule. Non-limiting examples of solid supports include any of beads, such as polystyrene beads and paramagnetic beads and derivatives thereof, affinity columns, microtitre plates etc. Where the substrate nucleic acid molecule is in fact two (or more) nucleic acid molecules which are ligated together, either one or both of the substrate nucleic acid molecules may be immobilized on a solid support. In specific embodiments, the separate substrate nucleic acid molecules may be immobilized on the same support as one another. This allows the molecules to be in proximity to ensure that ligation is efficient if the NAD-dependent ligase is present in the sample under test. Biotin and/or the streptavidin reagents may be incorporated in the kits to facilitate immobilisation for example.

In further embodiments, the kits further comprise, consist essentially of or consist of further reagents necessary for nucleic acid amplification. Preferably, the reagents are for carrying out any one of the amplification techniques discussed herein. Reagents for carrying out nucleic acid amplification are commercially available and well characterised in the art. The kits include suitable primers designed to amplify the novel ligated nucleic acid molecule. The discussion of primer design in respect of the methods of the invention applies *mutatis* mutandis here. Thus, suitable primers of the invention may be incorporate into suitable kits for detecting ligated nucleic acid molecules (e.g. as set forth in SEQ ID NOs: 5 and 6 or 9 and 10 respectively). Polymerases, such as Taq polymerase of which several variants (including hot start variants) are available and buffers such as KCl and (NH₄)₂SO₄ may also be included.

The kit may further comprise, consist essentially of or consist of reagents for detecting the products of nucleic acid amplification in real time or at end point. The kit may include reagents for carrying out real-time or end point amplification and detection utilising any of the fluorescence based systems disclosed herein. Suitable reagents for use in these methods are well known in the art and are commercially available.

Examples of suitable reagents include sequence specific probes. These probes may bind in between the primers used to amplify the novel nucleic acid molecule, and thus may bind to amplified nucleic acid molecules to provide a direct indicator of the levels of product being formed during amplification. The design of such probes is routine for one of skill in the art. Alternatively, appropriate primers may need to be designed, for example in the Amplifluour and Scorpion systems which allow real time or end point detection of amplification products. Thus, the kits of the invention may incorporate hairpin primers (Amplifluor), hairpin probes (Molecular Beacons), hydrolytic probes (Taqman), FRET probe pairs (Lightcycler), primers incorporating a hairpin probe (Scorpion), fluorescent dyes (SYBR Green etc.), primers incorporating the complementary sequence of a DNAzyme and a cleavable fluorescent DNAzyme substrate (DzYNA) or oligonucleotide blockers for example. A suitable hydrolytic probe is set forth as SEQ ID NO: 11.

Any suitable fluorophore is included within the scope of the invention for labelling, or as part of, the relevant primers or probes. Fluorophores that may possibly be included in the kits of the invention include, by way of example, FAM, HEX^{™}, NED^{™}, ROX^{™}, Texas Red^{™} etc. Similarly the kits of the invention are not limited to a single quencher. Quenchers, for example Dabcyl and TAMRA are well known quencher molecules that may be used in the method of the invention and included in the kits.

Kits may also include further components necessary for the generation and detection of PCR products other than those described above, such as microarrays, which may be used for detection of amplification products, or may be used to amplify (amplification on a chip) and detect the amplification product. Other components may further include "micro fluid cards" as described by Applied Biosystems, Reversed hybridization strips such as those described by LIPA technology (Innogenetics, Zwijnaarde, Belgium, or those described by Ulysis and ULS technology (Kreatech Biotechnologies, Amsterdam, The Netherlands)). Such components are known in the art and are listed by way of example and not limitation for inclusion in the kits.

The sample for testing with the kits may be any suitable sample, as defined above.

Additionally lysis reagents, which lyse other cells which are not target bacterial cells or micro-organisms but which may otherwise contribute ligase activity to the assay system may be included in the kits of the invention. Suitable reagents, which preferably do not lyse the cells of the bacteria or other micro-organisms which are to be detected include, by way of example and not limitation alcohols, salts etc and possibly also reagents such as proteinase K and chloroform depending upon which bacteria or other micro-organisms are being detected. In the specific application to detecting bacterial contamination of platelets, sodium carbonate and zwittergent may be utilised at an appropriate concentration to lyse platelets selectively and leave any bacterial cells in tact. Thus, the kits may incorporate these components.

The kits may further comprise, consist essentially of or consist of an enzyme for removing or exhausting from the sample ATP, to thus prevent any ATP dependent ligase activity in the sample influencing the bacterial detection. The enzyme may comprise any one or more of luciferase, phosphatase and pyrophosphatase, since all of these enzymes may be used to exhaust the ATP signal derived from non-target cells or organisms in the sample which may otherwise give rise to false positive results. Suitable reagents for these enzymes, such as an appropriate (storage) buffer may be included in the kits.

The kits may further comprise, consist essentially of or consist of reagents for lysis of the cells of the bacteria or other micro-organisms being detected in order to release the NAD-dependent ligase. Suitable reagents include by way of example and not limitation phenol, chloroform, proteinase K and lysostaphin, in particular lysotaphins disclosed herein. Agents for increasing cellular permeability, in order to permit detection of NAD-dependent ligase activity, may similarly be incorporated as discussed herein.

The kits may further comprise, consist essentially of or consist of one or more nucleases in order to degrade nucleic acid molecules associated with the bacteria or other micro-organisms which provide the NAD-dependent ligase activity which is detected in the sample. This may be beneficial to prevent non-specific ligation events for example. This may not be an absolute requirement however, since the substrate nucleic acid molecule may be designed such that they are not homologous to the nucleic acid molecules of the bacteria or other micro-organisms organism (whose viability is) being detected. Thus, specific detection of the novel ligated nucleic acid molecule can be achieved in the presence of "contaminating" endogenous nucleic acid. This is discussed in detail above, which discussion and embodiments apply *mutatis mutandis* to the kits of the invention.

As stated above, the kits may include means for selective removal of unligated substrate molecules. This helps to prevent unligated substrate influencing the sensitivity and/or specificity of detection of the ligated nucleic acid molecules. The means for selective removal of unligated substrate molecules may comprise one or more selective nucleases. In particular, one or more exonucleases may be employed to remove unligated substrate molecules. 3'-5' exonucleases such as ExoIII, ExoI and/or ExoT could be employed to digest unligated substrate molecules. A combination of dsDNA specific 3'-5' exonucleases, such as ExoIII and one or more ssDNA specific 3'-5' exonucleases such as ExoI and/or ExoT may advantageously be incorporated into the kits, in particular where the ligated nucleic acid molecule is formed from at least three substrate nucleic acid molecules which together form a double stranded region including a nick which is ligated by NAD-dependent ligase activity in the sample.

The kit may be provided with suitable buffers that allow all of the components to be provided in the same compartment or storage vessel. Thus, the complete kit is essentially provided as a complete (homogeneous) reaction mix. Thus, the methods of the invention may be carried out in a single reaction step which reduces the possibility of cross contamination of samples and also provides rapid results. Especially preferred is a reaction mix which provides results in real time or at end point. Preferably, such a reaction mix is an aqueous composition, but may be provided as a dry powder for reconstitution using sterile or distilled water for example.

Preferably, the reagents included allow an isothermal amplification technique to be utilised, such as TMA. The reagents may allow the isothermal amplification technique (such as TMA) to be carried out in real-time or at end point.

All kits may be provided with suitable instructions for use in any one of the methods of the invention. The instructions may be provided as an insert, for example as a booklet provided inside the packaging of the kit and/or may be printed on the packaging of the kit for example.

Also disclosed is a spray device for administering the reaction mix which contains all components necessary to carry out the methods of the invention to a surface. Any suitable spray device may be utilised, which may be a pump spray or an aerosolized device for example. Such a device may find application in a number of settings where microbial detection, or detection of any contaminating micro-organisms from any source, on a surface is required. For example, where food is being prepared it would be advantageous to be able to ensure that, following cleaning of surfaces after food preparation, no potentially harmful bacterial cells or micro-organisms remain on the surface. This ensures that the surface is then "clean" and may be utilised for further food preparation. A spray device can also be used to detect bacterial contamination on surfaces in a hygiene monitoring application, by detecting NAD-dependent ligase activity. Thus, the presence of this enzyme as a marker of (viable) bacteria indicates that some form of contamination is present on the surface. Use of an isothermal DNA amplification method is preferred for the detection of NAD-dependent ligase activity on a surface. Suitable examples such as TMA are referred to above.

The invention will be further defined by and understood with respect to the detailed description, incorporating the accompanying figures and examples in which:

### DETAILED DESCRIPTION OF THE INVENTION

### BRIEF DESCRIPTION OF THE FIGURES

Figure 1 is a schematic of the Ligase Mediated Assay of the invention that detects the bacterial enzyme, NAD-dependent ligase. NAD-dependent ligase is found exclusively in eubacteria (2) and has not been reported in mammals. Hence this technology is ideal for use in the rapid and sensitive detection of bacteria in clinical samples where background from the host would otherwise be a problem. An additional advantage of the methods of the invention is that there is a further amplification step in the system since NAD-dependent ligase generates many molecules of the DNA primer prior to NAT amplification. So this assay is a sensitive approach; currently the detection limit for E.coli and S. aureus is in the range 100 to 1000 cells in culture samples.
Figure 2 shows a plot of relative amplification versus time for various reaction conditions (with or without additional NAD+ and with or without gentamicin treatment).
Figure 3 is a flow chart showing the protocol for detection of Staph. aureus using the methods of the present invention.
Figure 4. Dilution of Staph. aureus cells. Detection using methods of the present invention (referred to as LiMA-2) and real time PCR (Taq-Man). From left to right the curves represent: 10⁶, 10⁵, 10⁴, 10³, 10², 10, 0 cells and the PCR blank.
Figure 5. Sensitivity of Staph. aureus cells to antibiotic. 10⁴ cells were added to culture medium, PCR traces are shown, from left to right: T=5 hours culture (no oxacillin), T=0 hours and T=5 hours culture (plus oxacillin)

### Example 1. Detection of platelet contamination

### Rationale.

Bacterial contamination of platelets can be detected by selective lysis of the platelets followed by bacterial cell lysis and detection of the bacterial ligase. Any contaminating mammalian ligase will not be detected efficiently by the assay because mammalian ligases use ATP as a cofactor whereas bacterial ligases use NAD+. The ligase buffer in this case is supplemented with the bacterial specific NAD+ cofactor. The bacterial ligase is detected by ligation of two synthetic DNA duplexes with a complementary 5 base overhang. Once ligated, ligated molecules are detected by PCR across the ligation junction.

### Method

1. 0.5 ml platelets (collected by apherisis) were spiked with known numbers of Escherichia coli, Staphylococcus aureus and Pseudomonas aeruginosa bacteria (asses by prior culture and enumeration).
2. The spiked samples were made 50mM sodium carbonate and 1% (w/v) Zwittergent in a final volume of 1 ml.
3. After incubation for 5 min to allow platelet lysis 100µl 4M Tris pH 7.5 was added.
4. The bacteria were pelleted by centrifugation at 6,000 x g for 5 min and the supernatant removed.
5. 20µl BPer (Pierce) was added and incubated 5 min to allow bacterial cell lysis.
6. 2µl of the bacterial lysate was then added to a ligase reaction containing 2µl 10x E.coli ligase buffer (NEB), 2µl (20ng) each of the DNA substrate S1/AS1 and S2/AS2 in a total volume of 20 µl. The DNA substrate is formed from 4 synthetic oligos which have a ligatable complementary 5 base overlap :
   S1 5' GCCGATATCGGACAACGGCCGAACTGGGAAGGCGCACGGAGAGA 3' (SEQ ID NO: 1)
   AS1 3' TATAGCCTGTTGCCGGCTTGACCCTTCCGCGTGCCTCTCTGGTGC 5', PHOSPHORYLATED AT THE 5' END (SEQ ID NO: 2)
   S2 5' CCACGAAGTACTAGCTGGCCGTTTGTCACCGACGCCTA 3', PHOSPHORYLATED AT THE 5' END (SEQ ID NO: 3)
   AS2 3' TTCATGATCGACCGGCAAACAGTGGCTGCGGAT 5' (SEQ ID NO: 4)

   The substrate was formed by mixing equimolar concentrations of S1 and AS1 at 93°C and allowing to cool to room temperature. Similarly, S2 and AS2 were formed in the same way.
7. After 30 min at room temperature 2 µl of the ligated product was investigated by PCR.
8. The real time PCR (Eurogentec) contained SYBR Green and the forward primer, 5' GGACAACGGCCGAACTGGGAAGG 3' (SEQ ID NO: 5) and reverse primer, 3' CGACCGGCAAACAGTGGCTGCGGAT 5' (SEQ ID NO: 6) with dentauration at 94°C for 10 sec, annealing at 65°C for 15 sec and extension at 72°C for 15 sec.

### Results

| Number of *E.coli* | Cycle at which PCR was positive |
|---|---|
| 10⁶ | 14.5 |
| 10⁵ | 18.44 |
| 10⁴ | 20.82 |
| 10³ | 23.88 |
| 10² | 25.55 |
| 10¹ | 27.95 |

| Number of *S. aureus* | Cycle at which PCR was positive |
|---|---|
| 10⁶ | 14.2 |
| 10⁵ | 17.55 |
| 10⁴ | 20.16 |
| 10³ | 22.65 |
| 10² | 25.74 |
| 10¹ | 28.55 |

| Number of P. *aeruginosa* | Cycle at which PCR was positive |
|---|---|
| 10⁶ | 15.42 |
| 10⁵ | 18.65 |
| 10⁴ | 21.18 |
| 10³ | 23.68 |
| 10² | 26.93 |
| 10¹ | 29.83 |
| no bact ctrl | 30.58 |
| No platelet ctrl | 30.48 |
| PCR ctrl | 34.26 |

### Discussion

Spiking with 10-fold dilutions of bacteria gave a corresponding titration of PCR signal. From the PCR results it can be seen that as few as 10 bacteria can be detected spiked into 0.5 ml of platelets. Similar results were achieved with replacing the sodium carbonate with ammonium sulphate for platelet lysis.

### Example 2. Drug susceptibility testing

### Rationale.

The PCR signal generated is related to the number of ligated molecules of the DNA substrate which in turn is related to the number of ligase molecules present. In turn, the number of molecules of ligase present is dependent on the viability of the bacterium. Upon culture of a given bacterium, if the bacteria are healthy, growing and increasing in number there will be progressively more ligase present which will generate more ligation and more PCR signal. If the bacteria are unhealthy and non-viable or dying there will be no increase in ligase on culture and the number of ligase molecules might even be expected to decrease which will be reflected in the amount of ligation and PCR signal generated. In this example we have tested the susceptibility of the organism to an antibiotic. By culturing in the presence and absence of antibiotic we can compare the signal generated from the bacterial ligase at different time points. Additionally, the experiment can be performed with and without the addition of the NAD+ cofactor. In the presence of added cofactor the ligase can cycle through many ligation steps. In the absence of added cofactor the ligase must depend on endogenous bacterial NAD+ for ligation. These two conditions, therefore are two different measures of the health of the bacterial cell

### Method

1. 10 ml of media (LB broth) containing 50µg/ml gentamicin was inoculated with 104 E.coli bacteria/ml and incubated for 3 hours. At 30 min time points 1 ml of the culture was removed and placed on ice until completion of the time course.
2. The bacteria were then pelleted by centrifugation at 6,000 x g for 5 min and the supernatant removed.
3. The protocol from this stage was identical to steps 3 to 8 in example 1 except that the ligase reaction was performed with and without added NAD+.

### Results

| | Cycle at which PCR was positive | | | |
|---|---|---|---|---|
| | With added NAD+ | | Without added NAD+ | |
| | Plus gentamicin | Minus gentamicin | Plus gentamicin | Minus gentamicin |
| Time (min) | | | | |
| 0 | 26.2 | 24.5 | 29.5 | 29.85 |
| 30 | 25.1 | 25 | 29.4 | 29.95 |
| 60 | 25.7 | 23.6 | 31.6 | 31.64 |
| 90 | 26.2 | 22.7 | 30.1 | 28.52 |
| 120 | 27.2 | 21 | 30.3 | 25.53 |
| 150 | 26.8 | 20.1 | 29.4 | 24.35 |
| 180 | 26.3 | 18.2 | 30.1 | 22.71 |
| No bacterial control | 29.3 | | 30.6 | |

The results can be presented as a graph in which the amplification factor at each time point and for each condition is plotted compared to the time 0 time point (see figure 2).

### Discussion

The assay works well as an antibiotic susceptibility test. In the presence of gentamicin the signal from the bacterial ligase does not increase with incubation. However, in the absence of gentamicin there is progressively more ligation and associated PCR signal at each time point. This reflects the growth and viability of the E.coli. In this example, only 104 E.coli was used as the original inoculum which demonstrates the high sensitivity of this approach. Inclusion of NAD+ in the ligation buffer allows the ligase enzyme to cycle through many molecules of DNA substrate and so the sensitivity of detection is increased. However, in the absence of exogenous NAD+, the bacterial enzyme must utilise bacterial NAD+ and although the signal is delayed there is a greater difference in signal between time 0 and the later time points. This reflects the fact that non-viable bacteria will have decreased levels of available NAD+.

### Example 3. Detection of bacterial ligase after nucleic acid capture

This example demonstrates that the bacterial ligase can be captured from solution using immobilised nucleic acid substrate. This example was essentially similar to example 1 but the nucleic acid substrate was immobilised onto streptavin beads through the synthesis of AS1 biotinylated at the 3' end.

### Method

1. Initially, the method was the same as steps 1-5 of example 1 using 0.5 ml platelets (collected by apherisis) and spiking with known numbers of Escherichia coli, Staphylococcus aureus and Pseudomonas aeruginosa bacteria (asses by prior culture and enumeration).
2. After bacterial cell lysis using 20µl BPer the solution was made up to 100µl with 1x ligase buffer which did not contain NAD+.
3. The nucleic acid duplex S1/AS1 was formed as described previously except that AS1 was biotinylated at the 3' end and the duplex bound to streptavidin beads (Sigma) at a concentration of 20ng of duplex per 20µl beads. 20µl beads with immobilised S1/AS1 was added to the lysate from step 2 above and incubated for 10 min.
4. The beads with attached ligase were collected using a magnet and placed into a 20µl ligation reaction including 1x ligase buffer with NAD+ and 20ng of the nucleic acid duplex S2/AS2.
5. After ligation for 30 min, the reaction was heated at 95°C for 5 min and 2µl placed into a PCR as described in example 1, step 8.

### Results

| Number of *E.coli* | Cycle at which PCR was positive |
|---|---|
| 10⁶ | 12.3 |
| 10⁵ | 15.36 |
| 10⁴ | 17.22 |
| 10³ | 20.15 |
| 10² | 22.89 |
| 10¹ | 25.67 |

| Number of *S. aureus* | Cycle at which PCR was positive |
|---|---|
| 10⁶ | 12.4 |
| 10⁵ | 14.57 |
| 10⁴ | 18.16 |
| 10³ | 21.35 |
| 10² | 23.99 |
| 10¹ | 26.15 |

| Number of *P. aeruginosa* | Cycle at which PCR was positive |
|---|---|
| 10⁶ | 12.92 |
| 10⁵ | 15.23 |
| 10⁴ | 18.64 |
| 10³ | 21.21 |
| 10² | 23.94 |
| 10¹ | 26.76 |
| no bact ctrl | 30.52 |
| No platelet ctrl | 30.39 |
| PCR ctrl | 34.10 |

### Discussion

This example shows that the ligase released from the bacteria can be captured onto an immobilised nucleic acid substrate prior to subsequent ligation. This approach concentrates the ligase and allows analysis of a greater proportion of the released ligase. This results in a more sensitive detection which is reflected in the PCR analysis for a given number of organisms becoming positive at an earlier cycle number.

### Example 4. NAD-dependent ligase detection : from Stap. aureus

### Media Wash Step:

Take 1ml fresh *Staph. aureus* culture (10⁶ cells /µl), spin 8000 rpm 5min, resuspend pellet in 1ml deionised water Dilute in a series of 10 fold dilutions down to 10² cells/µl

### Lysis /Ligation Mix

| | |
|---|---|
| Add 2µl "10x ligase buffer" | (4 mM MgCl2, 1mM DTT, 50 µg/ml BSA, 26µM NAD⁺, 30 mM Tris pH 8) |
| DNA substrate** ("anchor and template") | 1µl (annealed to 100ng/µl final concentration) |
| Heat treated lysostaphin | 2µl* |
| 1% Triton X-100 | 2µl |
| Non specific DNA 100µM | 0.2µl (single stranded oligomer) |
| Deionised water | 12.8µl |

| | |
|---|---|
| Add 1µl of relevant *Staph.* aureus culture dilution Incubate at room temperature for 30mins *Take stock lysostaphin, 10mg/ml, dilute 10x in 1x final ligase buffer without added NAD⁺, heat at 55 °C 5 min, then dilute 10x again. Note that heat treating the lysostaphin eliminates contaminating nucleases and ligases from the solution, so reducing background and loss of signal problems in the subsequent NAD-dependent ligase assay. ** Two components, which when hybridized generate a double stranded section with a nick. | |

Anchor: 5'-CCCCGGATCCCTTAGAATTCCCCTCAGAGGCACTGGAGCTGGAGACGTA-3' (SEQ ID NO: 7)
Template: 5'P-

### Treating unligated substrate

After ligation step above add 2µl of following nuclease mix to each tube:
10x dilution ExoIII 2µl
ExoI 2µl
ExoT 2µl
100x dilution of the "10x ligase buffer" (without NAD⁺) 14µl Incubate at 37 °C for 30mins
Heat to 95°C for 5mins (to inactivate the Exonucleases)

### PCR step

Add 2µl to PCR mastermix, cycles were:
90°C 10min 1x, followed by:
90°C 5sec 40x
60°C 10sec 40x

### Using:

Primer1 5'-GAGTCTACGAGTCTACGAGTTCT-3'(SED ID NO:9)
Primer2 5'-TCATCACTACGCCGAACTGC-3' (SEQ ID NO:10)
Taqman Probe 5'FAM-CTCAGAGGCACTGGAGCTGGAGAC-3'TAM (SEQ ID NO:11) Dual Labeled HPL (5' fluorescein and 3' TAMRA quencher)

### Results

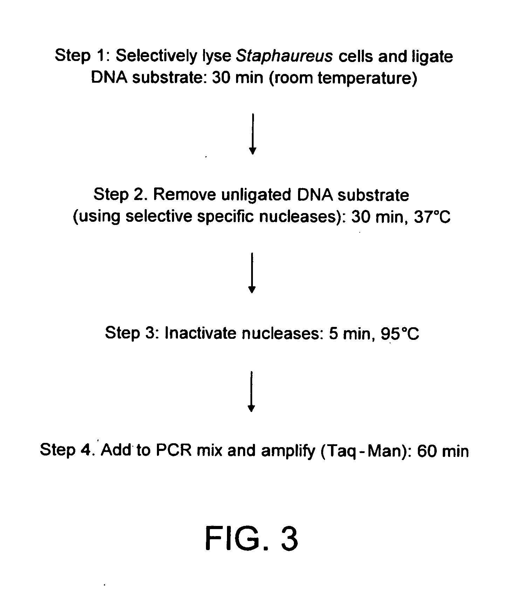

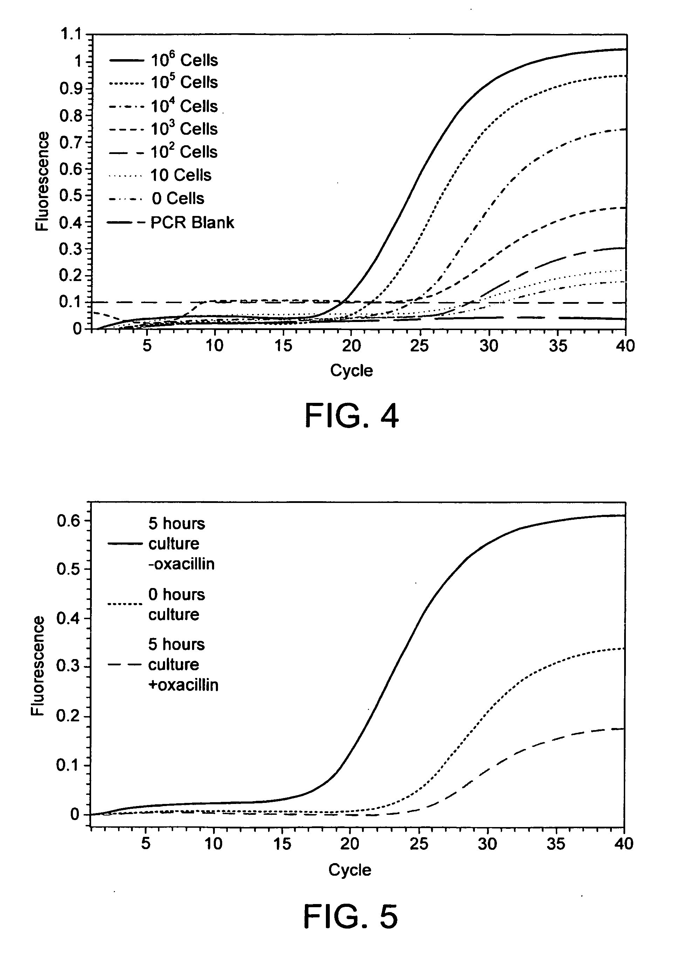

Figure 3 shows the assay protocol for the detection of Staph. aureus using the LiMA-2 technology. Figure 4 shows typical data from a serial dilution of Staph. aureus obtained with a 'real-time' PCR assay using the Taq-Man approach. The detection limit in this experiment was estimated to be between 10 and 100 cells. Figure 5 shows the effect of adding an antibiotic to the culture medium, the NAD-dependent ligase content falls by >2x10³ fold after 4 hours in the presence of antibiotic.

### Conclusion

We have shown that NAD-dependent ligase is a useful marker for viable bacterial cells and can be detected very sensitively with a nucleic acid template based technique. The very low detection limit for Staph. aureus indicates that the LiMA-2 technique is one of the most sensitive methods currently available for bacterial detection.

### References

Barringer KJ, Orgel L, Wahl G, Gingeras TR. Blunt-end and single-strand ligations by Escherichia coli ligase: influence on an in vitro amplification scheme. Gene. 1990 Apr 30:89(1):117-22.
Compton J. Nucleic acid sequence-based amplification. Nature. 1991 Mar 7;350(6313):91-2.
Fahy E, Kwoh DY, Gingeras TR. Self-sustained sequence replication (3SR): an isothermal transcription-based amplification system alternative to PCR.
   PCR Methods Appl. 1991 Aug;1(1):25-33. Review.
The LiMA Technology: Measurement of ATP on a Nucleic Acid Testing Platform. Banin S, Wilson SM and Stanley CJ (2007). Clinical Chemistry 53, 2034-2036
Recent developments in ligase-mediated amplification and detection. Cao W (2004). Trends in Biotechnology 22(1), 38-44

The present invention is not to be limited in scope by the specific embodiments described herein. Indeed, various modifications of the invention in addition to those described herein will become apparent to those skilled in the art from the foregoing description and accompanying figures. Such modifications are intended to fall within the scope of the appended claims. Moreover, all embodiments described herein are considered to be broadly applicable and combinable with any and all other consistent embodiments, as appropriate.

## Claims

1. A method of detecting the presence of an NAD-dependent ligase expressing micro-organism in a sample comprising:
(a) contacting the sample with a ligatable nucleic acid molecule which acts as a substrate for NAD-dependent ligase activity in the sample,
(b) incubating the thus contacted sample under conditions suitable for NAD-dependent ligase activity; and
(c) specifically determining the presence of a ligated nucleic acid molecule resulting from the action of the NAD-dependent ligase on the substrate nucleic acid molecule to indicate the presence of the NAD-dependent ligase expressing micro-organism; wherein the presence of a ligated nucleic acid molecule is determined using a nucleic acid amplification technique.

2. A method of screening for resistance of a bacterial cell or other NAD-dependent ligase expressing micro-organism to an agent directed against said cell, bacterium or other micro-organism, the method comprising the steps of, in a sample:
(a) exposing the bacterial cell or micro-organism to the agent;
(b) contacting the sample with a ligatable nucleic acid molecule which acts as substrate for NAD-dependent ligase activity in the sample,
(c) incubating the thus contacted sample under conditions suitable for NAD-dependent ligase activity; and
(d) specifically detecting whether there is present a ligated nucleic acid molecule resulting from the action of the NAD-dependent ligase on the substrate nucleic acid molecule wherein if there is resistance, the ligated nucleic acid molecule will be detected or will be detected at higher levels; wherein the presence of a ligated nucleic acid molecule is determined using a nucleic acid amplification technique.

3. A method of screening for agents which are capable of killing or preventing growth of one or more bacterial cells or other NAD-dependent ligase expressing micro-organisms, the method comprising the steps of, in a sample:
(a) exposing the bacterial cell or micro-organism to the agent;
(b) contacting the sample with a ligatable nucleic acid molecule which acts as substrate for NAD-dependent ligase activity in the sample,
(c) incubating the thus contacted sample under conditions suitable for NAD-dependent ligase activity; and
(d) specifically detecting whether there is present a ligated nucleic acid molecule resulting from the action of the NAD-dependent ligase on the substrate nucleic acid molecule, wherein if the agent is capable of killing or preventing growth of the bacterium or micro-organism the novel nucleic acid molecule will not be detected or will be detected at lower levels; wherein the presence of a ligated nucleic acid molecule is determined using a nucleic acid amplification technique.

4. A method of claim 1 used for diagnosing an infection, or a disease associated with the presence of a bacterial cell or other NAD-dependent ligase expressing micro-organism in a subject, comprising the steps of, in a sample obtained from the subject:
(a) contacting the sample with a ligatable nucleic acid molecule which acts as a substrate for NAD-dependent ligase activity in the sample,
(b) incubating the thus contacted sample under conditions suitable for NAD-dependent ligase activity; and
(c) specifically determining the presence of a ligated nucleic acid molecule resulting from the action of the NAD-dependent ligase on the substrate nucleic acid molecule to indicate the presence of the bacterium or other micro-organism as an indication of infection or disease; wherein the presence of a ligated nucleic acid molecule is determined using a nucleic acid amplification technique.

5. A method of claim 1 used for detecting the presence of bacterial contamination in a platelet containing sample comprising:
(a) contacting the platelet sample with a ligatable nucleic acid molecule which acts as a substrate for NAD-dependent ligase activity in the sample,
(b) incubating the thus contacted sample under conditions suitable for NAD-dependent ligase activity; and
(c) specifically determining the presence of a ligated nucleic acid molecule resulting from the action of the NAD-dependent ligase on the substrate nucleic acid molecule to indicate the presence of the bacterial contamination in the platelet containing sample; wherein the presence of a ligated nucleic acid molecule is determined using a nucleic acid amplification technique.

6. A method for determining the presence of bacteria of interest, which are resistant to a specific anti-bacterial agent, in a sample comprising:
(a) incubating the bacteria of interest in an incubating medium including the specific anti-bacterial agent,
(b) capturing the incubated bacteria of interest using a specific capture reagent,
(c) exposing the incubated bacteria of interest to an agent capable of causing lysis of the bacteria or of increasing the permeability of the bacterial cell wall to a degree such that the presence of NAD-dependent ligase from the bacteria can be determined, and
(d) determining the presence of the (resistant) bacteria of interest in the sample by:
(i) contacting the sample with a ligatable nucleic acid molecule which acts as a substrate for NAD-dependent ligase activity in the sample,
(ii) incubating the thus contacted sample under conditions suitable for NAD-dependent ligase activity; and
(iii) specifically determining the presence of a ligated nucleic acid molecule resulting from the action of the NAD-dependent ligase on the substrate nucleic acid molecule to indicate the presence of the (NAD-dependent ligase expressing) bacteria of interest; wherein the presence of a ligated nucleic acid molecule is determined using a nucleic acid amplification technique.

7. The method of any preceding claim wherein the ligatable nucleic acid molecule which acts as substrate for NAD-dependent ligase activity in the sample is utilised in molar excess over NAD-dependent ligase, if present, in the sample.

8. The method of any preceding claim wherein the nucleic acid molecule which acts as substrate for NAD-dependent ligase activity lacks nucleotide sequence identity with the genomic nucleic acid of the NAD-dependent ligase expressing micro-organism to ensure specificity of detection of the ligated nucleic acid molecule.

9. The method of any preceding claim wherein the conditions suitable for NAD-dependent ligase activity include supplying the sample with additional NAD.

10. The method of any of claims 1 to 8 which relies upon endogenous NAD+ to support NAD-dependent ligase activity.

11. The method of any preceding claim which further comprises selective lysis of the NAD-dependent ligase expressing micro-organism in the sample to release NAD-dependent ligase.

12. The method of any preceding claim which further comprises capture of NAD-dependent ligase from the sample.

13. Use of NAD-dependent ligase activity as an indicator of the presence of an NAD-dependent ligase expressing micro-organism in a sample; wherein the presence of a ligated nucleic acid molecule is determined using a nucleic acid amplification technique.

## Patentansprüche

1. Verfahren zum Nachweis der Gegenwart eines eine NAD-abhängige Ligase exprimierenden Mikroorganismus in einer Probe, das umfasst:
(a) Inkontaktbringen der Probe mit einem ligierbaren Nucleinsäuremolekül, das als Substrat für eine NAD-abhängige Ligaseaktivität in der Probe dient;
(b) Inkubieren der auf diese Weise in Kontakt gebrachten Probe unter Bedingungen, die für eine NAD-abhängige Ligaseaktivität geeignet sind; und
(c) gezieltes Bestimmen der Gegenwart eines ligierten Nucleinsäuremoleküls, das durch die Einwirkung der NAD-abhängigen Ligase auf das als Substrat dienende Nucleinsäuremolekül entstanden ist, um die Gegenwart des eine NAD-abhängige Ligase exprimierenden Mikroorganismus anzuzeigen, wobei die Gegenwart eines ligierten Nucleinsäuremoleküls unter Verwendung einer Nucleinsäure-Amplifikationstechnik ermittelt wird.

2. Verfahren zum Screening auf die Resistenz einer Bakterienzelle oder eines anderen, eine NAD-abhängige Ligase exprimierenden Mikroorganismus gegenüber einem gegen die Zelle, das Bakterium oder den anderen Mikroorganismus gerichteten Stoff, wobei das Verfahren Schritte umfasst, in einer Probe:
(a) die Bakterienzelle oder den Mikroorganismus dem Stoff auszusetzen;
(b) die Probe mit einem ligierbaren Nucleinsäuremolekül in Kontakt zu bringen, das als Substrat für eine NAD-abhängige Ligaseaktivität in der Probe dient;
(c) die auf diese Weise in Kontakt gebrachte Probe unter Bedingungen, die für eine NAD-abhängige Ligaseaktivität geeignet sind, zu inkubieren; und
(d) gezielt zu bestimmen, ob ein ligiertes Nucleinsäuremolekül vorhanden ist, das durch die Einwirkung der NAD-abhängigen Ligase auf das als Substrat dienende Nucleinsäuremolekül entstanden ist, wobei das ligierte Nucleinsäuremolekül nachgewiesen oder in größeren Mengen nachgewiesen wird, wenn eine Resistenz vorhanden ist, und wobei die Gegenwart eines ligierten Nucleinsäuremoleküls unter Verwendung einer Nucleinsäure-Amplifikationstechnik ermittelt wird.

3. Verfahren zum Screening auf Stoffe, die befähigt sind, eine oder mehrere Bakterienzellen oder andere eine NAD-abhängige Ligase exprimierende Mikroorganismen abzutöten oder deren Wachstum zu unterbinden, wobei das Verfahren Schritte umfasst, in einer Probe:
(a) die Bakterienzelle oder den Mikroorganismus dem Stoff auszusetzen;
(b) die Probe mit einem ligierbaren Nucleinsäuremolekül in Kontakt zu bringen, das als Substrat für eine NAD-abhängige Ligaseaktivität in der Probe dient;
(c) die auf diese Weise in Kontakt gebrachte Probe unter Bedingungen, die für eine NAD-abhängige Ligaseaktivität geeignet sind, zu inkubieren; und
(d) gezielt zu bestimmen, ob ein ligiertes Nucleinsäuremolekül vorhanden ist, das durch die Einwirkung der NAD-abhängigen Ligase auf das als Substrat dienende Nucleinsäuremolekül entstanden ist, wobei das neue Nucleinsäuremolekül nicht nachgewiesen oder auf niedrigerem Niveau nachgewiesen wird, wenn der Stoff befähigt ist, das Bakterium oder den Mikroorganismus abzutöten oder deren Wachstum zu unterbinden; wobei die Gegenwart eines ligierten Nucleinsäuremoleküls unter Verwendung einer Nucleinsäure-Amplifikationstechnik ermittelt wird.

4. Verfahren nach Anspruch 1, das zur Diagnose einer Infektion oder Erkrankung eingesetzt wird, die mit der Gegenwart einer Bakterienzelle oder eines anderen eine NAD-abhängige Ligase exprimierenden Mikroorganismus bei einer Testperson in Zusammenhang steht, wobei das Verfahren Schritte umfasst, in einer von der Testperson erhaltenen Probe:
(a) die Probe mit einem ligierbaren Nucleinsäuremolekül in Kontakt zu bringen, das als Substrat für eine NAD-abhängige Ligaseaktivität in der Probe dient;
(b) die auf diese Weise in Kontakt gebrachte Probe unter Bedingungen, die für eine NAD-abhängige Ligaseaktivität geeignet sind, zu inkubieren; und
(c) die Gegenwart eines ligierten Nucleinsäuremoleküls gezielt zu bestimmen, das durch die Einwirkung der NAD-abhängigen Ligase auf das als Substrat dienende Nucleinsäuremolekül entstanden ist, um die Gegenwart des Bakteriums oder anderen Mikroorganismus als Hinweis auf die Infektion oder Erkrankung anzuzeigen; wobei die Gegenwart eines ligierten Nucleinsäuremoleküls unter Verwendung einer Nucleinsäure-Amplifikationstechnik ermittelt wird.

5. Verfahren nach Anspruch 1, das zum Nachweis des Vorhandenseins einer bakteriellen Kontaminierung in einer Thrombozyten enthaltenden Probe eingesetzt wird, das umfasst:
(a) die Thrombozytenprobe mit einem ligierbaren Nucleinsäuremolekül in Kontakt zu bringen, das als Substrat für eine NAD-abhängige Ligaseaktivität in der Probe dient;
(b) die auf diese Weise in Kontakt gebrachte Probe unter Bedingungen, die für eine NAD-abhängige Ligaseaktivität geeignet sind, zu inkubieren; und
(c) die Gegenwart eines ligierten Nucleinsäuremoleküls gezielt zu bestimmen, das durch die Einwirkung der NAD-abhängigen Ligase auf das als Substrat dienende Nucleinsäuremolekül entstanden ist, um das Vorhandenseins einer bakteriellen Kontaminierung in der Thrombozyten enthaltenden Probe anzuzeigen; wobei die Gegenwart eines ligierten Nucleinsäuremoleküls unter Verwendung einer Nucleinsäure-Amplifikationstechnik ermittelt wird.

6. Verfahren zum Nachweis der Gegenwart eines Bakteriums von Interesse, das gegenüber einem speziellen antibakteriellen Stoff resistent ist, in einer Probe, das umfasst:
(a) das Bakterium von Interesse in einem Inkubationsmedium, das den speziellen antibakteriellen Stoff enthält, zu inkubieren;
(b) die inkubierten Bakterien von Interesse unter Verwendung eines speziellen Abfangmittels abzufangen;
(c) die inkubierten Bakterien von Interesse einem Stoff auszusetzen, der befähigt ist, die Lyse der Bakterien hervorzurufen oder die Permeabilität der Bakterienzellwand soweit zu erhöhen, dass die Gegenwart einer NAD-abhängigen Ligase der Bakterien bestimmt werden kann;
(d) die Gegenwert der (resistenten) Bakterien von Interesse in der Probe zu bestimmen, durch
(i) Inkontaktbringen der Probe mit einem ligierbaren Nucleinsäuremolekül, das als Substrat für eine NAD-abhängige Ligaseaktivität in der Probe dient;
(ii) Inkubieren der auf diese Weise in Kontakt gebrachten Probe unter Bedingungen, die für eine NAD-abhängige Ligaseaktivität geeignet sind; und
(iii) gezieltes Bestimmen der Gegenwart eines ligierten Nucleinsäuremoleküls, das durch die Einwirkung der NAD-abhängigen Ligase auf das als Substrat dienende Nucleinsäuremolekül entstanden ist, um die Gegenwart des (eine NAD-abhängige Ligase exprimierenden) Bakteriums von Interesse anzuzeigen, wobei die Gegenwart eines ligierten Nucleinsäuremoleküls unter Verwendung einer Nucleinsäure-Amplifikationstechnik ermittelt wird.

7. Verfahren nach einem der vorhergehenden Ansprüche, wobei das ligierbare Nucleinsäuremolekül, das als Substrat für eine NAD-abhängige Ligaseaktivität in der Probe dient, in Bezug auf die NAD-abhängige Ligase, falls vorhanden, in der Probe in molarem Überschuss verwendet wird.

8. Verfahren nach einem der vorhergehenden Ansprüche, wobei das ligierbare Nucleinsäuremolekül, das als Substrat für eine NAD-abhängige Ligaseaktivität dient, keine Nucleotidsequenzidentität mit der genomischen Nucleinsäure des die NAD-abhängige Ligase exprimierenden Mikroorganismus aufweist, um den spezifischen Nachweis des ligierten Nucleinsäuremoleküls sicher zu stellen.

9. Verfahren nach einem der vorhergehenden Ansprüche, wobei die Bedingungen, die für eine NAD-abhängige Ligaseaktivität geeignet sind, umfassen, die Probe mit weiterem NAD zu versorgen.

10. Verfahren nach einem der Ansprüche 1 bis 8, das sich zur Unterstützung der NAD-abhängigen Ligaseaktivität auf endogenes NAD+ stützt.

11. Verfahren nach einem der vorhergehenden Ansprüche, das ferner die selektive Lyse des die NAD-abhängige Ligase exprimierenden Mikroorganismus in der Probe umfasst, um NAD-abhängige Ligase freizusetzen.

12. Verfahren nach einem der vorhergehenden Ansprüche, das ferner das Abfangen der NAD-abhängigen Ligase aus der Probe umfasst.

13. Verwendung von NAD-abhängiger Ligaseaktivität als Indikator für die Gegenwart eines eine NAD-abhängige Ligase exprimierenden Mikroorganismus in einer Probe, wobei die Gegenwart eines ligierten Nucleinsäuremoleküls unter Verwendung einer Nucleinsäure-Amplifikationstechnik ermittelt wird.

## Revendications

1. Procédé pour détecter la présence d'un microorganisme exprimant une ligase dépendant du NAD dans un échantillon, comprenant les étapes consistant :
(a) à mettre en contact l'échantillon avec une molécule d'acide nucléique apte à la ligation, qui joue le rôle de substrat pour l'activité de ligase dépendant du NAD dans l'échantillon,
(b) à mettre en incubation l'échantillon ainsi mis en contact dans des conditions convenables pour l'activité de ligase dépendant du NAD ; et
(c) à déterminer spécifiquement la présence d'une molécule d'acide nucléique jointe par ligation, résultant de l'action de la ligase dépendant du NAD sur la molécule d'acide nucléique servant de substrat, pour indiquer la présence du microorganisme exprimant la ligase dépendant du NAD ; dans lequel la présence d'une molécule d'acide nucléique jointe par ligation est déterminée en utilisant une technique d'amplification d'acides nucléiques.

2. Procédé pour déterminer la résistance d'une cellule bactérienne ou d'un autre microorganisme exprimant une ligase dépendant du NAD à un agent dirigé contre ladite cellule, ladite bactérie ou ledit autre microorganisme, le procédé comprenant les étapes consistant à, dans un échantillon :
(a) exposer la cellule bactérienne ou le microorganisme à l'agent ;
(b) mettre en contact l'échantillon avec une molécule d'acide nucléique apte à la ligation, qui joue le rôle de substrat pour l'activité de ligase dépendant du NAD dans l'échantillon,
(c) mettre en incubation l'échantillon ainsi mis en contact dans des conditions convenables pour l'activité de ligase dépendant du NAD ; et
(d) détecter spécifiquement si est présente une molécule d'acide nucléique jointe par ligation, résultant de l'action de la ligase dépendant du NAD sur la molécule d'acide nucléique servant de substrat, dans lequel, s'il existe une résistance, la molécule d'acide nucléique jointe par ligation sera détectée ou bien sera détectée à des taux plus élevés ; la présence d'une molécule d'acide nucléique jointe par ligation étant déterminée en utilisant une technique d'amplification d'acides nucléiques.

3. Procédé pour déterminer des agents qui sont capables de tuer, ou d'empêcher la croissance d'une ou plusieurs cellules bactériennes ou d'autres microorganismes exprimant une ligase dépendant du NAD, le procédé comprenant les étapes consistant à, dans un échantillon :
(a) exposer la cellule bactérienne ou le microorganisme à l'agent ;
(b) mettre en contact l'échantillon avec une molécule d'acide nucléique apte à la ligation, qui joue le rôle de substrat pour l'activité de ligase dépendant du NAD dans l'échantillon,
(c) mettre en incubation l'échantillon ainsi mis en contact dans des conditions convenables pour l'activité de ligase dépendant du NAD ; et
(d) détecter spécifiquement si est présente une molécule d'acide nucléique jointe par ligation, résultant de l'action de la ligase dépendant du NAD sur la molécule d'acide nucléique servant de substrat, dans lequel, si l'agent est capable de tuer ou d'empêcher la croissance de la bactérie ou du microorganisme, la molécule d'acide nucléique nouvelle ne sera pas détectée ou bien sera détectée à des taux réduits ; la présence d'une molécule d'acide nucléique jointe par ligation étant déterminée en utilisant une technique d'amplification d'acides nucléiques.

4. Procédé suivant la revendication 1, utilisé pour diagnostiquer une infection, ou une maladie associée à la présence d'une cellule bactérienne ou d'un autre microorganisme exprimé en ligase dépendant du NAD chez un sujet, comprenant les étapes consistant à, dans un échantillon obtenu à partir du sujet :
(a) mettre en contact l'échantillon avec une molécule d'acide nucléique apte à la ligation, qui joue un rôle de substrat pour l'activité de ligase dépendant du NAD dans l'échantillon,
(b) mettre en incubation l'échantillon ainsi mis en contact dans des conditions convenables pour l'activité de ligase dépendant du NAD ; et
(c) déterminer spécifiquement la présence d'une molécule d'acide nucléique jointe par ligation, résultant de l'action de la ligase dépendant du NAD sur la molécule d'acide nucléique servant de substrat pour indiquer la présence de la bactérie ou de l'autre microorganisme comme indication d'une infection ou d'une maladie ; la présence d'une molécule d'acide nucléique jointe par ligation étant déterminée en utilisant une technique d'amplification d'acides nucléiques.

5. Procédé suivant la revendication 1, utilisé pour détecter la présence d'une contamination bactérienne dans un échantillon contenant des plaquettes, comprenant l'étape consistant :
(a) à mettre en contact l'échantillon contenant des plaquettes avec une molécule d'acide nucléique apte à la ligation, qui joue le rôle de substrat pour l'activité de ligase dépendant du NAD dans l'échantillon,
(b) à mettre en incubation l'échantillon ainsi mis en contact dans des conditions convenables pour l'activité de ligase dépendant du NAD ; et
(c) à déterminer spécifiquement la présence d'une molécule d'acide nucléique jointe par ligation, résultant de l'action dans la ligase dépendant du NAD sur la molécule d'acide nucléique servant de substrat, pour indiquer la présence de la contamination bactérienne dans l'échantillon contenant des plaquettes ; la présence d'une molécule d'acide nucléique jointe par ligation étant déterminée en utilisant une technique d'amplification d'acides nucléiques.

6. Procédé pour déterminer la présence de bactéries intéressantes, qui sont résistantes à un agent anti-bactérien spécifique, dans un échantillon, comprenant les étapes consistant :
(a) à mettre en incubation les bactéries intéressantes dans un milieu d'incubation comprenant l'agent anti-bactérien spécifique,
(b) à capturer la bactérie intéressante qui a été mise en incubation, en utilisant un réactif de capture spécifique,
(c) à exposer les bactéries intéressantes ayant été mises en incubation à un agent capable de provoquer la lyse des bactéries ou d'augmenter la perméabilité dans la paroi des cellules bactériennes à un degré tel que la présence d'une ligase dépendant du NAD des bactéries puissent être déterminées, et
(d) à déterminer la présence des bactéries intéressantes (résistantes) dans l'échantillon :
(i) en mettant en contact l'échantillon avec une molécule d'acide nucléique apte à la ligation qui joue le rôle de substrat pour l'activité de ligase dépendant du NAD dans l'échantillon,
(ii) en mettant en incubation l'échantillon ainsi mis en contact dans des conditions convenables pour l'activité de ligase dépendant du NAD ; et
(iii) en déterminant spécifiquement la présence d'une molécule d'acide nucléique jointe par ligation résultant de l'action de la ligase dépendant du NAD sur la molécule d'acide nucléique servant de substrat pour indiquer la présence des bactéries intéressantes (exprimant la ligase dépendant du NAD) ; dans lequel la présence d'une molécule d'acide nucléique jointe par ligation est déterminée en utilisant une technique d'amplification d'acides nucléiques.

7. Procédé suivant l'une quelconque des revendications précédentes, dans lequel la molécule d'acide nucléique jointe par ligation qui joue le rôle de substrat pour l'activité de ligase dépendant du NAD de l'échantillon est utilisée en un excès molaire par rapport à la ligase dépendant du NAD, s'il est présent, dans l'échantillon.

8. Procédé suivant l'une quelconque des revendications précédentes, dans lequel la molécule d'acide nucléique qui joue le rôle de substrat pour l'activité de ligase dépendant du NAD est dépourvue d'identité de séquence de nucléotides avec l'acide nucléique génomique du microorganisme exprimant la ligase dépendant du NAD pour garantir la spécificité de détection de la molécule d'acide nucléique jointe par ligation.

9. Procédé suivant l'une quelconque des revendications précédentes, dans lequel les conditions convenables pour l'activité de ligase dépendant du NAD comprennent l'apport d'une quantité de NAD supplémentaire à l'échantillon.

10. Procédé suivant l'une quelconque des revendications 1 à 8, qui est basé sur le NAD plus endogène pour entretenir l'activité de ligase dépendant du NAD.

11. Procédé suivant l'une quelconque des revendications précédentes, qui comprend en outre une lyse sélective du microorganisme exprimant une ligase dépendant du NAD dans l'échantillon pour libérer la ligase dépendant du NAD.

12. Procédé suivant l'une quelconque des revendications précédentes, qui comprend en outre la capture de la ligase dépendant du NAD de l'échantillon.

13. Utilisation d'une activité de ligase dépendant du NAD comme indicateur de la présence d'un microorganisme exprimant une ligase dépendant du NAD dans un échantillon ; dans lequel la présence d'une molécule d'acide nucléique jointe par ligation est déterminée en utilisant une technique d'amplification d'acides nucléiques.
